# EUROPEAN PATENT APPLICATION

(11) **EP 2 308 482 A1**
(43) Date of publication of application: **13.04.2011**
(21) Application number: 10185770.4
(22) Date of filing: 18.08.2006
(51) Int. Cl.: A61K 31/165, A61P 21/00, A61P 19/00

(54) **Novel use of peptide compounds for treating muscle pain**

(30) Priority: 08.06.2006 US 811840 P; 08.06.2006 US 811859 P; 18.08.2005 EP 05017977
(62) Divisional of application: 06776964.6
(71) Applicant: UCB Pharma GmbH, 40789 Monheim (DE)
(72) Inventor: Beyreuther, Bettina, 40219, Düsseldorf (DE); Stöhr, Thomas, 40789, Monheim (DE)
(74) Representative: Weiss, Wolfgang

(57) **Abstract**

The present invention is directed to a pharmaceutical composition comprising a class of peptide compounds suitable for treating non-inflammatory musculoskeletal pain or/and non-inflammatory osteoarthritic pain.

## Description

The present invention is directed to the use of a class of peptide compounds for the treatment of non-inflammatory musculoskeletal pain or/and non-inflammatory osteoarthritic pain.

Certain peptides are known to exhibit central nervous system (CNS) activity and are useful in the treatment of epilepsy and other CNS disorders. These peptides which are described in the U.S. Patent No. 5,378,729 have the Formula (Ia): 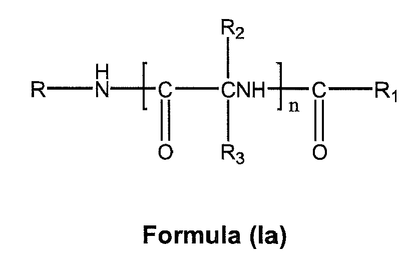 wherein
R is hydrogen, lower alkyl, lower alkenyl, lower alkynyl, aryl, aryl lower alkyl, heterocyclic, heterocyclic lower alkyl, lower alkyl heterocyclic, lower cycloalkyl, lower cycloalkyl lower alkyl, and R is unsubstituted or is substituted with at least one electron withdrawing group or electron donating group;
R₁ is hydrogen or lower alkyl, lower alkenyl, lower alkynyl, aryl lower alkyl, aryl, heterocyclic lower alkyl, heterocyclic, lower cycloalkyl, lower cycloalkyl lower alkyl, each unsubstituted or substituted with an electron donating group or an electron withdrawing group; and
R₂ and R₃ are independently hydrogen, lower alkyl, lower alkenyl, lower alkynyl, aryl lower alkyl, aryl, heterocyclic, heterocyclic lower alkyl, lower alkyl heterocyclic, lower cycloalkyl, lower cycloalkyl lower alkyl, or Z-Y wherein R₂ and R₃ may be unsubstituted or substituted with at least one electron withdrawing group or electron donating group;
Z is O, S, S(O)ₐ, NR₄, PR₄ or a chemical bond;
Y is hydrogen, lower alkyl, aryl, aryl lower alkyl, lower alkenyl, lower alkynyl, halo, heterocyclic, heterocyclic lower alkyl, and Y may be unsubstituted or substituted with an electron donating group or an electron withdrawing group, provided that when Y is halo, Z is a chemical bond, or
ZY taken together is NR₄NR₅R₇, NR₄OR₅, ONR₄R₇, OPR₄R₅, PR₄OR₅, SNR₄R₇, NR₄SR₇, SPR₄R₅ or PR₄SR₇, NR₄PR₅R₆ or PR₄NR₅R₇, 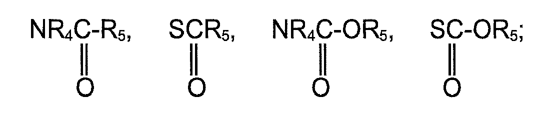 R₄, R₅ and R₆ are independently hydrogen, lower alkyl, aryl, aryl lower alkyl, lower alkenyl, or lower alkynyl, wherein R₄, R₅ and R₆ may be unsubstituted or substituted with an electron withdrawing group or an electron donating group; and
R₇ is R₆ or COOR₈ or COR₈;
R₈ is hydrogen or lower alkyl, or aryl lower alkyl, and the aryl or alkyl group may be unsubstituted or substituted with an electron withdrawing group or an electron donating group; and
n is 1-4; and
a is 1-3.

U.S. Patent No. 5,773,475 also discloses additional compounds useful for treating CNS disorders. These compounds are N-benzyl-2-amino-3-methoxy-propionamide having the Formula (IIa): 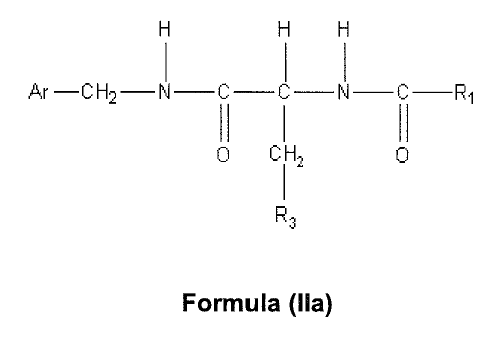 wherein

Ar is aryl which is unsubstituted or substituted with halo; R₃ is lower alkoxy; and R₁ is methyl.

The patents US 5.378.729 and US 5.773.475 are hereby incorporated by reference. However, neither of these patents describes the use of these compounds for treating specific manifestations of non-inflammatory musculoskeletal pain such as muscular hyperalgesia and allodynia occurring in fibromyalgia, myofascial pain syndrome, back pain or osteoarthritis.

WO 02/074297 relates to the use of a compound according to Formula (IIa) wherein Ar is phenyl which may be substituted by at least one halo, R₃ is lower alkoxy containing 1-3 carbon atoms and R₁ is methyl for the preparation of pharmaceutical compositions useful for the treatment of allodynia related to peripheral neuropathic pain.

WO 02/074784 relates to the use of a compound having Formula (Ia) or/and Formula (IIa) showing antinociceptive properties for treating different types and symptoms of acute and chronic pain, especially non neuropathic inflammatory pain, e.g. rheumatoid arthritic pain or/and secondary inflammatory osteo-arthritic pain.

Non-inflammatory musculoskeletal pain is a specific form of pain. Non-inflammatory musculoskeletal pain is clearly distinguished from pain induced by tissue damage and macrophage infiltration (resulting in edema) as classical immune system response.

Non-inflammatory musculoskeletal pain which is not traced to a specific structural or inflammatory cause fits the classification criteria for fibromyalgia syndrome (FMS), myofascial pain syndrome (MPS) or back pain. It is believed that non-inflammatory musculoskeletal pain results from peripheral and central sensitization (Staud 2002). The knowledge on involved basic mechanisms, animal models to assess muscle pain and treatment regimens need to be improved.

Fibromyalgia is a complex syndrome associated with significant impairment on the quality of life and function and substantial financial costs (10). Fibromyalgia is also referred to herein as fibromyalgia syndrome (FMS).

Fibromyalgia is a systemic process that causes tender points (local tender areas in normal-appearing tissues) in typical areas of the body and is frequently associated with a poor sleep pattern and stressful environment. The diagnosis of fibromyalgia is based on the history of widespread pain, defined as bilateral, upper and lower body, as well as spine, and the presence of excessive tenderness on applying pressure to at least 11 of 18 specific muscletendon sites. Fibromyalgia is typically a chronic syndrome that causes pain and stiffness throughout the tissue that support and move the bones and joints.

The treatment of fibromyalgia is conventionally based on pain-relievers, NSAIDs, muscle relaxants, tranquilizers and anti-depressant drugs, whereby none are particularly helpful. Fibromyalgia patients often sleep poorly and may experience some relief by taking the antidepressant amitriptyline at bedtime (JAMA. 2004 Nov 17; 292(19):2388-95. Management of fibromyalgia syndrome. Goldenberg DL, Burckhardt C, Crofford L.). The goal in treating fibromyalgia is to decrease pain and to increase function.

Myofascial pain syndrome (MPS) describes a chronic non-degenerative, non-inflammatory musculoskeletal pain condition. Distinct areas within muscles or their delicate connective tissue coverings (fascia) become abnormally thickened or tight. When the myofascial tissues tighten and lose their elasticity, neurotransmitter ability to send and receive messages between the brain and body is damaged. Symptoms include muscle stiffness and aching and sharp shooting pains or tingling and numbness in areas distant from the trigger point. The discomfort may cause sleep disturbance, fatigue, and depression. Most commonly trigger points are in the neck, back or buttocks.

Myofascial pain differs from fibromyalgia: myofascial pain syndromes and fibromyalgia are separate entities, each having its own pathology, but sharing the muscle as their common pathway of pain. Myofascial pain is a more localized or regional pain (along the muscle and surrounding fascia tissues) process that is associated with trigger point tenderness. Myofascial pain can be treated with a variety of methods (sometimes in combination) including stretching, ultrasound, ice sprays with stretching, exercises, and injections of anesthetic.

A further non-inflammatory musculoskeletal pain syndrome is back pain, notably low back pain. Back pain is a common musculoskeletal symptom that may be either acute or chronic. It may be caused by a variety of diseases and disorders that affect the lumbar spine. Low back pain is often accompanied by sciatica, which is pain that involves the sciatic nerve and is felt in the lower back, the buttocks, and the backs of the thighs.

Non-inflammatory musculoskeletal pain such as fibromyalgia, myofascial pain syndrome and back pain involve increased muscle sensitivity as an important manifestation. Increased muscle sensitivity is characterized by pain evoked by a normally non-nociceptive stimulus (allodynia) or increased pain intensity evoked by nociceptive stimuli (hyperalgesia).

Osteoarthritis is an acquired musculoskeletal disorder that is believed to be non-inflammatory in origin, occurring when the rate of cartilage degradation exceeds that of regeneration, resulting in cartilage erosion, subchondral bone thickening, and joint damage (Wieland, 2005). As cartilage thins, its surface integrity can be lost, clefts can form, and the cartilage tends to be more easily eroded with joint motion. As new cartilage is formed, it tends to be more fibrous and less able to withstand mechanical stress. Over time, underlying bone can be exposed that is less capable of withstanding mechanical stress, resulting in microfractures. Localized osteonecrosis can occur beneath the bone surface, leading to cysts that can further weaken the bone's support of the cartilage.

As osteoarthritis progresses, it can eventually influence structures surrounding the joint. Local inflammation such as synovitis can occur, for example in response to inflammatory mediators released during the cartilage degradation process. The joint capsule tends to thicken, and movement of nutrients into and metabolic waste products out of the joint can be restricted. Eventually, periarticular muscle wasting can become evident as osteoarthritis progresses, and the joint is used less often or improperly. Pain of osteoarthritis is thought to be due not to cartilage degradation per se but to effects on surrounding structures including bone, since cartilage is aneural.

Subchondral bone, periosteum, synovium, ligaments, and the joint capsule are all richly innervated and contain nerve endings that could be source of nociceptive stimuli (Heppelmann, 1997; Mach et al., 2002). In addition to peripheral pain sensitization, central pain sensitization can occur in osteoarthritis (Schaible et al., 2002).

According to the Centers for Disease Control and Prevention (CDC), osteoarthritis is the most common form of arthritic disease, affecting 21 million Americans. See http://www.cdc.gov/arthritis/data_statistics/arthritis_ related_statistics.htm#2.

By 2020, it is estimated that 60 million Americans will suffer from arthritis. Arthritis is the leading cause of physical disability (defined broadly as needing assistance in walking or climbing stairs) and of restricted daily activity in more than 7 million Americans, and this number is expected to grow to more than 11.6 million by 2020. See http://www.arthritis.org/ resources/ActionPlanlnterior.pdf.

It is very costly to treat arthritis and its complications. In 1997, the total cost of arthritis and other rheumatic conditions in the United States was $86 billion. The direct medical costs of arthritis and other rheumatic conditions in 1997 were $51.1 billion. The indirect costs (due to lost wages) of arthritis and other rheumatic conditions in 1997 were $35.1 billion. See http://www.cdc.gov/mmwr/preview/mmwrhtml/mm5318a3.htm.

The prevalence of osteoarthritis increases with age, and age is the largest risk factor. A survey reported by Brandt (2001) Principles of Internal Medicine, 15th ed. (Braunwald et al., eds.), New York: McGraw-Hill, pp. 1987-1994, found that only 2% of women less than 45 years old had radiographic evidence of osteoarthritis. In women aged 45 to 64 years, however, the prevalence was 30%, and for those 65 years or older it was 68%. Other risk factors include excess body weight, genetics, estrogen deficiency, repetitive joint use, and trauma.

A typical patient with osteoarthritis is middle-aged or elderly and complains of pain in the knee, hip, hand or spine. The distal and proximal interphalangeal joints of the hands are the most common sites of osteoarthritis but also the least likely to be exhibit symptoms. The hip and knee are the second and third most common joints seen on X-ray to be affected, with knee pain being more likely to exhibit symptoms.

Pain is the paramount symptom of osteoarthritis. Osteoarthritic pain can have one or both of an inflammatory and a non-inflammatory component. Anti-inflammatory agents such as NSAIDs (non-steroidal anti-inflammatory drugs) and cyclooxygenase-2 inhibitors can be useful in treating or managing the inflammatory component, while opioid and other analgesics can be useful in treating or managing the non-inflammatory component. However, such drug therapies are not always effective and have side-effects that may not be well tolerated in all patients.

Non-inflammatory pain, in particular non-inflammatory musculoskeletal pain or/and non-inflammatory osteoarthritic pain, is often characterized by absence of swelling or warmth, absence of inflammatory or systemic features, and minimal or no morning stiffness.

Non-inflammatory osteoarthritic pain can contribute to a sedentary lifestyle, depression and sleep problems, particularly in the elderly. The pain is often characterized as a deep, aching sensation that intensifies with motion. It is usually intermittent and often mild, but can become persistent and severe. Crepitus is usually noted in the affected joints.

Non-inflammatory osteoarthritic pain is a specific type of non-inflammatory musculoskeletal pain which typically arises from effects of osteoarthritis-related morphological alterations, such as cartilage degradation, bone changes on sensory neurons, and vascularization of bone remodeling. It is distinguished herein from inflammatory osteoarthritic pain, which typically occurs from synovial inflammation following pathological processes in cartilage and bone involving tissue damage and macrophage infiltration (resulting in edema) associated with a classical immune system response.

A need exists to identify treatments having therapeutic efficacy in the treatment, in particular systemic treatment, of specific manifestations of non-inflammatory musculoskeletal pain or/and non-inflammatory osteoarthritic pain such as muscular hyperalgesia and allodynia occurring in fibromyalgia, myofascial pain syndrome, back pain or osteoarthritis.

Therefore, it is the problem of the invention to provide a treatment for non-inflammatory musculoskeletal pain or/and non-inflammatory osteoarthritic pain, in particular fibromyalgia, myofascial pain syndrome (MPS) or back pain. Specifically, it is a problem of the invention to provide a treatment, preferably a systemic treatment, of non-inflammatory musculoskeletal pain or/and non-inflammatory osteoarthritic pain including fibromyalgia, myofascial pain syndrome (MPS) or back pain which are characterized by increased pain intensity evoked by nociceptive stimuli (hyperalgesia) or by increased pain intensity evoked by normally non-nociceptive stimuli (allodynia) in the absence of a physiological cause such as inflammatory edema.

The development of second-generation antiepileptic drugs has created unprecedented opportunities for the treatment of chronic pain. These drugs modulate pain transmission by interacting with specific ion channels. The actions of antiepileptic drugs differ in neuropathic and non-neuropathic pain, and agents within each medication class have varying degrees of efficacy. First-generation antiepileptic drugs (i.e., carbamazepine, phenytoin) and second-generation antiepileptic drugs (e.g., gabapentin, pregabalin) are effective in the treatment of neuropathic pain. The efficacy of antidepressants and antiepileptic drugs in the treatment of neuropathic pain is comparable; tolerability also is comparable, but safety and side effect profiles differ. Tricyclic antidepressants are the most cost-effective agents, but second-generation antiepileptic drugs are associated with fewer safety concerns in elderly patients. Tricyclic antidepressants have documented (although limited) efficacy in the treatment of fibromyalgia and chronic low back pain.

Lacosamide (also called SPM 927 or Harkoseride) has a novel mode of action which is unknown insofar (Bialer et al., 2002). The mode of action of the compounds of Formulae (Ib) or/and (IIb) differs from that of common antiepileptic drugs. Ion channels are not affected by the compounds of the present invention in a manner comparable to other known antiepileptic drugs, whereas GABA-induced currents are potentiated, but no direct interaction with any known GABA receptor subtype is observed. Glutamate induced currents are attenuated but the compounds do not directly interact with any known glutamate receptor subtype.

Pressure hyperalgesia and TNF-induced reduction in grip force may be used as an animal model for non-inflammatory musculoskeletal pain. In humans, reduced grip strength is strongly associated with muscle pain. Indeed, alpha-and gamma-motorneurons in agonist muscles are inhibited after noxious chemical stimulation (6, 7, 8).

It was shown that TNF-induced reduction in grip force is indeed a measure of hyperalgesia rather than the consequence of muscle weakness, fatigue or disruption of the contractile apparatus. Rotarod testing indicates no motor impairment after TNF injection, and muscle histology showed no abnormalities (1). Withdrawal thresholds to pressure applied percutaneously to muscle were markedly reduced after TNF injection in most rats. This primary hyperalgesia parallels tenderness to palpation that is observed clinically in patients with myalgia, such as myofascial pain syndrome and fibromyalgia (3), which is a primary criterion for the diagnosis of muscle pain under clinical and experimental human conditions (4,5).

Since pain on palpation of muscles without morphological abnormalities is typical of the fibromyalgia syndrome in humans (2) the model of intramuscular injection of TNF may be used as a model of muscle pain related e.g. to fibromyalgia. Intramuscular injection of tumor necrosis factor-alpha (TNF) induces mechanical hyperalgesia in rats. This can be quantified by measuring the withdrawal threshold to muscle pressure and the grip strength. TNF injections do not lead to morphological damage of the muscle (1).

Using the model of TNF-injection into the muscle, it has now been found that lacosamide is effective in reducing antinociceptive behavior. Surprisingly, a complete reversal of TNF-induced muscle hyperalgesia in the gastrocnemius muscle was seen with lacosamide at 30 mg/kg and metamizol at 2 mg/kg. In biceps muscle hyperalgesia, a significant reversal of hyperalgesia was seen with lacosamide at 10 mg/kg and 30 mg/kg. A significant reduction of muscular hyperalgesia was also seen for pregabalin and gabapentin at 100 mg/kg and metamizol at 2 mg/kg.

One of the best characterized rat models for osteoarthritis is the injection of the metabolic inhibitor, monosodium iodoacetate, into the joint which inhibits the activity of glyceraldehyde-3-phosphate dehydrogenase in chondrocytes, resulting in disruption of the glycolysis and eventually in cell death (Guzman et al., 2003; Kalbhen, 1987). The progressive loss of chondrocytes results in histological and morphological changes of the articular cartilage, closely resembling those seen in human osteoarthritis patients.

Using the iodoacetate rat model of osteoarthritic pain, it was surprisingly found that lacosamide inhibited mechanical hyperalgesia during the post-inflammatory period, indicating effectiveness of lacosamide for treating non-inflammatory osteoarthritic pain.

The use of compounds of Formula (Ib) or/and Formula (IIb) for treatment of non-inflammatory musculoskeletal pain or/and non-inflammatory osteoarthritic pain has not been reported. Thus, the present invention concerns the use of said compounds of Formulae (Ib) or/and (IIb) for the preparation of a pharmaceutical composition for the prevention, alleviation or/and treatment of non-inflammatory musculoskeletal pain or/and non-inflammatory osteoarthritic pain, in particular specific manifestations of non-inflammatory musculoskeletal pain or/and non-inflammatory osteoarthritic pain such as muscular hyperalgesia or/and allodynia occurring in fibromyalgia, myofascial pain syndrome, back pain or/and osteoarthritis.

In the context of the present invention, allodynia includes muscular and non-muscular allodynia. It is preferred that allodynia is muscular allodynia.

Various pathological conditions may be responsible for non-inflammatory musculoskeletal pain or/and non-inflammatory osteoarthritic pain. Therefore, in the present invention, non-inflammatory musculoskeletal pain or/and non-inflammatory osteoarthritic pain also includes non-inflammatory musculoskeletal pain or/and non-inflammatory osteoarthritic pain associated with or/and caused by a pathological condition. Preferably, this condition is selected from regional pain syndrome such as back or neck pain, rheumatoid arthritis, osteoarthritis, gout, ankylosing spondylitis, lupus erythematosus, fibromyalgia, fibrositis, fibromyositis, myofascial pain syndrome, autoimmune disorders, polymyalgia rheumatica, polymyositis, dermatomyositis, muscular abscess, trichinosis, Lyme disease, Malaria, Rocky Mountain spotted fever, polio, trauma, joint damage, joint damage by trauma, cartilage degradation, structural bone changes, and vascularization of areas of osteoarthritic bone remodeling.

As used herein, the term "non-inflammatory osteoarthritic pain" refers to non-inflammatory pain associated with or/and caused by osteoarthritis. In particular, "non-inflammatory osteoarthritic pain" refers to non-inflammatory musculoskeletal pain associated with or/and caused by osteoarthritis.

In one embodiment of the present invention, the non-inflammatory musculoskeletal pain is non-inflammatory pain associated with or/and caused by osteoarthritis. In another embodiment of the present invention, the non-inflammatory musculoskeletal pain is non-inflammatory musculoskeletal pain associated with or/and caused by osteoarthritis.

It is also preferred that in the present invention, the non-inflammatory osteoarthritic pain is non-inflammatory osteoarthritic pain associated with or/and caused by a pathological condition selected from trauma, joint damage, joint damage by trauma, cartilage degradation, structural bone changes, and vascularization of areas of osteoarthritic bone remodeling. Bone continually undergoes remodeling. Remodeling is a process in which old bone is replaced with new bone to maintain peak bone density. Vascularization occurs by the proliferation of capillaries during the remodeling process and can be increased in conditions such as osteoarthritis.

It is also preferred that in the present invention, the non-inflammatory pain, in particular the the non-inflammatory musculoskeletal pain or/and non-inflammatory osteoarthritic pain is characterized by the absence of swelling or warmth, absence of inflammatory or/and systemic features, or/and essentially no morning stiffness.

Since cartilage is aneural, osteoarthritic pain can be mediated by effects of cartilage degradation on surrounding structures, such as bone and joint capsule. In the present invention, cartilage degradation includes, but is not limited to cartilage erosion, loss of surface integrity, cleft formation, and increased erosion with joint motion.

In the present invention, structural bone changes include, but are not limited to subchrondral bone thickening, microfractures, osteonecrosis, osteonecrosis beneath bone surface, and weakend bone support of the cartilage.

In the present invention, osteoarthritic pain includes, but is not limited to osteoarthritic pain in the knee, hip, hand, or/and spine.

Non-inflammatory musculoskeletal pain or/and non-inflammatory osteoarthritic pain may be responsible for a number of symptoms, which may be remedied or at least relieved by the treatment of the non-inflammatory musculoskeletal pain or/and the non-inflammatory osteoarthritic pain. Therefore, in the present invention, non-inflammatory musculoskeletal pain or/and non-inflammatory osteoarthritic pain further includes a condition associated with or/and caused by non-inflammatory musculoskeletal pain or/and non-inflammatory osteoarthritic pain. Preferably, this condition is selected from fatigue, sleep disorder, irritable bowel syndrome, chronic headache, temporo-mandibular joint dysfunction syndrome, multiple chemical sensitivity, painful menstrual periods, dysmenorrhea, chest pain, morning stiffness, cognitive or memory impairment, numbness and tingling sensations, muscle twitching, irritable bladder, the feeling of swollen extremities, skin sensitivities, dry eyes and mouth, frequent changes in eye prescription, dizziness and impaired coordination.

As used herein, the term "non-inflammatory arthritic pain" refers to non-inflammatory pain associated with or/and caused by arthritis or/and a condition secondary to arthritis. In particular, "non-inflammatory arthritic pain" refers to non-inflammatory musculoskeletal pain associated with or/and caused by arthritis or/and a condition secondary to arthritis such as osteoarthritis.

Pain related to arthritis, for example in osteoarthritis, can be inflammatory or non-inflammatory or both. In one embodiment of the present invention, the non-inflammatory musculoskeletal pain is non-inflammatory pain associated with or/and caused by arthritis or/and a condition secondary to arthritis, such as osteoarthritis.

In another embodiment of the present invention, the non-inflammatory musculoskeletal pain is non-inflammatory musculoskeletal pain associated with or/and caused by arthritis or/and a condition secondary to arthritis, such as osteoarthritis.

An "arthritic condition" or "arthritis" as used herein is a musculoskeletal disorder, usually accompanied by pain, of one or more joints of a subject, and includes arthritis associated with or secondary to conditions that are not necessarily primarily arthritic. Among the most important arthritic conditions is osteoarthritis, which can be idiopathic or primary in origin, or secondary to other conditions.

A compound according to the invention has the general Formula (Ib) 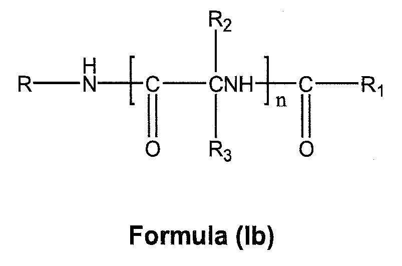 wherein
R is hydrogen, lower alkyl, lower alkenyl, lower alkynyl, aryl, aryl lower alkyl, heterocyclic, heterocyclic lower alkyl, lower alkyl heterocyclic, lower cycloalkyl or lower cycloalkyl lower alkyl, and R is unsubstituted or is substituted with at least one electron withdrawing group, and/or at least one electron donating group;
R₁ is hydrogen or lower alkyl, lower alkenyl, lower alkynyl, aryl lower alkyl, aryl, heterocyclic lower alkyl, lower alkyl heterocyclic, heterocyclic, lower cycloalkyl, lower cycloalkyl lower alkyl, each unsubstituted or substituted with at least one electron donating group and/or at least one electron withdrawing group;
and
R₂ and R₃ are independently hydrogen, lower alkyl, lower alkenyl, lower alkynyl, aryl lower alkyl, aryl, halo, heterocyclic, heterocyclic lower alkyl, lower alkyl heterocyclic, lower cycloalkyl, lower cycloalkyl lower alkyl, or Z-Y wherein R₂ and R₃ may be unsubstituted or substituted with at least one electron withdrawing group and/or at least one electron donating group;
Z is O, S, S(O)ₐ, NR₄, NR'₆, PR₄ or a chemical bond;
Y is hydrogen, lower alkyl, aryl, aryl lower alkyl, lower alkenyl, lower alkynyl, halo, heterocyclic, heterocyclic lower alkyl, lower alkyl heterocyclic and Y may be unsubstituted or substituted with at least one electron donating group and/or at least one electron withdrawing group, provided that when Y is halo, Z is a chemical bond, or
ZY taken together is NR₄NR₅R₇, NR₄OR₅, ONR₄R₇, OPR₄R₅, PR₄OR₅, SNR₄R₇, NR₄SR₇, SPR₄R₅, PR₄SR₇, NR₄PR₅R₆, PR₄NR₅R₇ or N⁺R₅R₆R₇, 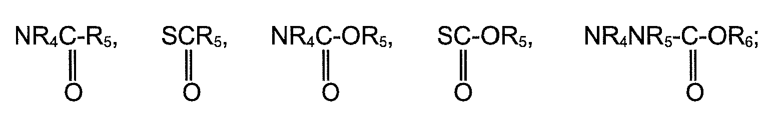 R'₆ is hydrogen, lower alkyl, lower alkenyl, or lower alkenyl which may be unsubstituted or substituted with at least one electron withdrawing group or/and at least one electron donating group;
R₄, R₅ and R₆ are independently hydrogen, lower alkyl, aryl, aryl lower alkyl, lower alkenyl, or lower alkynyl, wherein R₄, R₅ and R₆ may independently be unsubstituted or substituted with at least one electron withdrawing group or/and at least one electron donating group;
R₇ is R₆ or COOR₈ or COR₈, which R₇ may be unsubstituted or substituted with at least one electron withdrawing group or/and at least one electron donating group;
R₈ is hydrogen or lower alkyl, or aryl lower alkyl, and the aryl or alkyl group may be unsubstituted or substituted with at least one electron withdrawing group or/and at least one electron donating group; and
n is 1-4; and
a is 1-3.

Preferably the compound according has the general Formula (IIb) 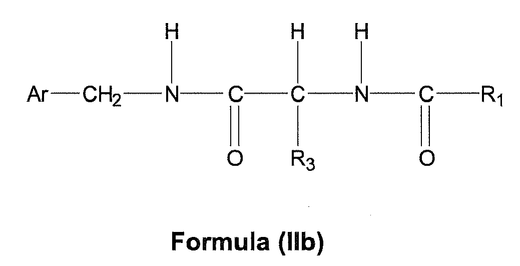 wherein

Ar is aryl, especially phenyl, which is unsubstituted or substituted with at least one halo; R₃ is -CH₂-Q, wherein Q is lower alkoxy; and R₁ is lower alkyl, especially methyl.

The present invention is also directed to a pharmaceutical composition comprising a compound according to Formula (Ib) or/and Formula (IIb) useful for the prevention, alleviation or/and treatment of muscle pain, in particular of muscle pain associated with or/and caused by a pathological condition, or/and of a condition associated with or/and caused by muscle pain.

The "lower alkyl" groups when used alone or in combination with other groups, are lower alkyl containing from 1 to 6 carbon atoms, especially 1 to 3 carbon atoms, and may be straight chain or branched. These groups include methyl, ethyl, propyl, isopropyl, butyl, isobutyl, tertiary butyl, amyl, hexyl, and the like.

The "lower alkoxy" groups are lower alkoxy containing from 1 to 6 carbon atoms, especially 1 to 3 carbon atoms, and may be straight chain or branched. These groups include methoxy, ethoxy, propoxy, butoxy, isobutoxy, tert-butoxy, pentoxy, hexoxy and the like.

The "aryl lower alkyl" groups include, for example, benzyl, phenylethyl, phenylpropyl, phenylisopropyl, phenylbutyl, diphenylmethyl, 1,1-diphenylethyl, 1,2-diphenylethyl, and the like.

The term "aryl", when used alone or in combination, refers to an aromatic group which contains from 6 up to 18 ring carbon atoms and up to a total of 25 carbon atoms and includes the polynuclear aromatics. These aryl groups may be monocyclic, bicyclic, tricyclic or polycyclic and are fused rings. A polynuclear aromatic compound as used herein, is meant to encompass bicyclic and tricyclic fused aromatic ring systems containing from 10-18 ring carbon atoms and up to a total of 25 carbon atoms. The aryl group includes phenyl, and the polynuclear aromatics e.g., naphthyl, anthracenyl, phenanthrenyl, azulenyl and the like. The aryl group also includes groups like ferrocenyl. Aryl groups may be unsubstituted or mono or polysubstituted with electron withdrawing or/and electron donating groups as described below.

The term "monosubstituted amino," alone or in combination with another term(s), means an amino substituent wherein one of the hydrogen radicals is replaced by a non-hydrogen substituent. The term "disubstituted amino," alone or in combination with another term(s), means an amino substituent wherein both of the hydrogen atoms are replaced by non-hydrogen substituents, which may be identical or different. An example of a monosubstituted amino substiuent is the N-alkoxyamino group. An example of a disubstituted amino substiuent is the N-alkoxy-N-alkylamino group.

"Lower alkenyl" is an alkenyl group containing from 2 to 6 carbon atoms and at least one double bond. These groups may be straight chained or branched and may be in the Z or E form. Such groups include vinyl, propenyl, 1-butenyl, isobutenyl, 2-butenyl, 1-pentenyl, (Z)-2-pentenyl, (E)-2-pentenyl, (Z)-4-methyl-2-pentenyl, (E)-4-methyl-2-pentenyl, pentadienyl, e.g., 1, 3 or 2,4-pentadienyl, and the like.

The term "lower alkynyl" is an alkynyl group containing 2 to 6 carbon atoms and may be straight chained as well as branched. It includes such groups as ethynyl, propynyl, 1-butynyl, 2-butynyl, 1-pentynyl, 2-pentynyl, 3-methyl-1-pentynyl, 3-pentynyl, 1-hexynyl, 2-hexynyl, 3-hexynyl and the like.

The term "lower cycloalkyl" when used alone or in combination is a cycloalkyl group containing from 3 to 18 ring carbon atoms and up to a total of 25 carbon atoms. The cycloalkyl groups may be monocyclic, bicyclic, tricyclic, or polycyclic and the rings are fused. The cycloalkyl may be completely saturated or partially saturated. Examples include cyclopropyl, cyclobutyl, cyclopentyl, cyclohexyl, cycloheptyl, cyclooctyl, cyclodecyl, cyclohexenyl, cyclopentenyl, cyclooctenyl, cycloheptenyl, decalinyl, hydroindanyl, indanyl, fenchyl, pinenyl, adamantyl, and the like. Cycloalkyl includes the cis or trans forms. Cycloalkyl groups may be unsubstituted or mono or polysubstituted with electron withdrawing or/and electron donating groups as described below. Furthermore, the substituents may either be in endo or exo positions in the bridged bicyclic systems.

The term "electron-withdrawing and electron donating" refer to the ability of a substituent to withdraw or donate electrons, respectively, relative to that of hydrogen if the hydrogen atom occupied the same position in the molecule. These terms are well understood by one skilled in the art and are discussed in Advanced Organic Chemistry, by J. March, John Wiley and Sons, New York, NY, pp.16-18 (1985) and the discussion therein is incorporated herein by reference. Electron withdrawing groups include halo, including bromo, fluoro, chloro, iodo and the like; nitro, carboxy, lower alkenyl, lower alkynyl, formyl, carboxyamido, aryl, quaternary ammonium, haloalkyl such as trifluoromethyl, aryl lower alkanoyl, carbalkoxy and the like. Electron donating groups include such groups as hydroxy, lower alkoxy, including methoxy, ethoxy and the like; lower alkyl, such as methyl, ethyl, and the like; amino, lower alkylamino, di(loweralkyl) amino, aryloxy such as phenoxy, mercapto, lower alkylthio, lower alkylmercapto, disulfide (lower alkyldithio) and the like. One of ordinary skill in the art will appreciate that some of the aforesaid substituents may be considered to be electron donating or electron withdrawing under different chemical conditions. Moreover, the present invention contemplates any combination of substituents selected from the above-identified groups.

The term "halo" or "halogen" includes fluoro, chloro, bromo, iodo and the like.

The term "carbalkoxy" refers to -CO-O-alkyl, wherein alkyl may be lower alkyl as defined above.

The prefix "halo" indicates that the substituent to which the prefix is attached is substituted with one or more independently selected halogen radicals. For example, haloalkyl means an alkyl substituent wherein at least one hydrogen radical is replaced with a halogen radical. Examples of haloalkyl substituents include chloromethyl, 1-bromoethyl, fluoromethyl, difluoromethyl, trifluoromethyl, 1,1,1-trifluoroethyl, and the like. Illustrating further, "haloalkoxy" means an alkoxy substituent wherein at least one hydrogen radical is replaced by a halogen radical. Examples of haloalkoxy substituents include chloromethoxy, 1-bromoethoxy, fluoromethoxy, difluoromethoxy, trifluoromethoxy (also known as "perfluoromethyloxy"), 1,1,1,-trifluoroethoxy, and the like. It should be recognized that if a substituent is substituted with more than one halogen radical, those halogen radicals may be identical or different, unless otherwise stated.

The term "acyl" includes lower alkanoyl containing from 1 to 6 carbon atoms and may be straight chains or branched. These groups include, for example, formyl, acetyl, propionyl, butyryl, isobutyryl, tertiary butyryl, pentanoyl and hexanoyl.

As employed herein, a heterocyclic group contains at least one sulfur, nitrogen or oxygen ring atom, but also may include several of said atoms in the ring. The heterocyclic groups contemplated by the present invention include heteroaromatics and saturated and partially saturated heterocyclic compounds. These heterocyclics may be monocyclic, bicyclic, tricyclic or polycyclic and are fused rings. They may preferably contain up to 18 ring atoms and up to a total of 17 ring carbon atoms and a total of up to 25 carbon atoms. The heterocyclics are also intended to include the so-called benzoheterocyclics. Representative heterocyclics include furyl, thienyl, pyrazolyl, pyrrolyl, methylpyrrolyl, imidazolyl, indolyl, thiazolyl, oxazolyl, isothiazolyl, isoxazolyl, piperidyl, pyrrolinyl, piperazinyl, quinolyl, triazolyl, tetrazolyl, isoquinolyl, benzofuryl, benzothienyl, morpholinyl, benzoxazolyl, tetrahydrofuryl, pyranyl, indazolyl, purinyl, indolinyl, pyrazolindinyl, imidazolinyl, imadazolindinyl, pyrrolidinyl, furazanyl, N-methylindolyl, methylfuryl, pyridazinyl, pyrimidinyl, pyrazinyl, pyridyl, epoxy, aziridino, oxetanyl, azetidinyl, the N-oxides of the nitrogen containing heterocycles, such as the N-oxides of pyridyl, pyrazinyl, and pyrimidinyl and the like. Heterocyclic groups may be unsubstituted or mono or polysubstituted with electron withdrawing or/and electron donating groups.

The preferred heterocyclics are thienyl, furyl, pyrrolyl, benzofuryl, benzothienyl, indolyl, methylpyrrolyl, morpholinyl, pyridiyl, pyrazinyl, imidazolyl, pyrimidinyl, or pyridazinyl. The preferred heterocyclic is a 5 or 6-membered heterocyclic compound. The especially preferred heterocyclic is furyl, pyridyl, pyrazinyl, imidazolyl, pyrimidinyl, or pyridazinyl. The most preferred heterocyclics are furyl and pyridyl.

In another preferred embodiment the heterocyclic is selected from furyl, furyl substituted by at least one lower alkyl group preferably containing 1-3 carbon atoms such as methyl, pyrrolyl, imidazolyl, pyridyl, pyrazinyl, pyrimidinyl, oxazolyl, and thiazolyl, more preferably from furyl, pyridyl, pyrazinyl, pyrimidinyl, oxazolyl, and thiazolyl, and most preferably from furyl, pyridyl, pyrimidinyl, and oxazolyl.

The preferred compounds are those wherein n is 1, but di (n=2), tri (n=3) and tetrapeptides (n=4) are also contemplated to be within the scope of the invention.

The preferred values of R is aryl lower alkyl, especially benzyl especially those wherein the phenyl ring thereof is unsubstituted or substituted with electron donating groups or/and electron withdrawing groups, such as halo (e.g., F).

The preferred R₁ is H or lower alkyl. The most preferred R₁ group is methyl.

The preferred electron donating substituents or/and electron withdrawing substituents are halo, nitro, alkanoyl, formyl, arylalkanoyl, aryloyl, carboxyl, carbalkoxy, carboxamido, cyano, sulfonyl, sulfoxide, heterocyclic, guanidine, quaternary ammonium, lower alkenyl, lower alkynyl, sulfonium salts, hydroxy, lower alkoxy, lower alkyl, amino, lower alkylamino, di(loweralkyl)amino, amino lower alkyl, mercapto, mercaptoalkyl, alkylthio, and alkyldithio. The term "sulfide" encompasses mercapto, mercapto alkyl and alkylthio, while the term disulfide encompasses alkyldithio. Especially preferred electron donating or/and electron withdrawing groups are halo or lower alkoxy, most preferred are fluoro or methoxy. These preferred substituents may be present on any one of the groups in Formula (Ib) or/and (IIb), e.g. R, R₁, R₂, R₃, R₄, R₅, R₆, R'₆, R₇, R₈, and/or R₅₀ as defined herein.

The ZY groups representative of R₂ and R₃ include hydroxy, alkoxy, such as methoxy, ethoxy, aryloxy, such as phenoxy; thioalkoxy, such as thiomethoxy, thioethoxy; thioaryloxy such as thiophenoxy; amino; alkylamino, such as methylamino, ethylamino; arylamino, such as anilino; lower dialkylamino, such as, dimethylamino; trialkyl ammonium salt, hydrazino; alkylhydrazino and arylhydrazino, such as N-methylhydrazino, N-phenylhydrazino, carbalkoxy hydrazino, aralkoxycarbonyl hydrazino, aryloxycarbonyl hydrazino, hydroxylamino, such as N-hydroxylamino (-NH-OH), lower alkoxy amino [(NHOR₁₈) wherein R₁₈ is lower alkyl], N-lower alkylhydroxyl amino [(NR₁₈)OH wherein R₁₈ is lower alkyl], N-lower alkyl-O-lower alkylhydroxyamino, i.e., [N(R₁₈)OR₁₉ wherein R₁₈ and R₁₉ are independently lower alkyl], and o-hydroxylamino (-O-NH₂); alkylamido such as acetamido; trifluoroacetamido; lower alkoxyamino, (e.g., NH(OCH₃); and heterocyclicamino, such as pyrazoylamino.

The preferred heterocyclic groups representative of R₂ and R₃ are monocyclic 5- or 6-membered heterocyclic moieties of the formula: 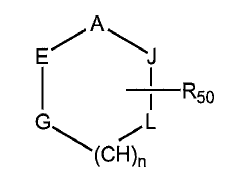 or those corresponding partially or fully saturated form thereof wherein n is 0 or 1; and
R₅₀ is H or an electron withdrawing group or electron donating group;
A, E, L, J and G are independently CH, or a heteroatom selected from the group consisting of N, O, S;
but when n is 0, G is CH, or a heteroatom selected from the group consisting of NH, O and S with the proviso that at most two of A, E, L, J and G are heteroatoms.

When n is 0, the above heteroaromatic moiety is a five membered ring, while if n is 1, the heterocyclic moiety is a six membered monocyclic heterocyclic moiety. The preferred heterocyclic moieties are those aforementioned heterocyclics which are monocyclic.

If the ring depicted hereinabove contains a nitrogen ring atom, then the N-oxide forms are also contemplated to be within the scope of the invention.

When R₂ or R₃ is a heterocyclic of the above formula, it may be bonded to the main chain by a ring carbon atom. When n is 0, R₂ or R₃ may additionally be bonded to the main chain by a nitrogen ring atom.

Other preferred moieties of R₂ and R₃ are hydrogen, aryl, e.g., phenyl, aryl alkyl, e.g., benzyl and alkyl.

It is to be understood that the preferred groups of R₂ and R₃ may be unsubstituted or mono or poly substituted with electron donating or/and electron withdrawing groups. It is preferred that R₂ and R₃ are independently hydrogen, lower alkyl, which is either unsubstituted or substituted with electron withdrawing groups or/and electron donating groups, such as lower alkoxy (e.g., methoxy, ethoxy, and the like), N-hydroxylamino, N-lower alkylhydroxyamino, N-loweralkyl-O-loweralkyl and alkylhydroxyamino.

It is preferred that one of R₂ and R₃ is hydrogen.

It is preferred that n is one.

It is more prefered that n=1 and one of R₂ and R₃ is hydrogen. It is especially preferred that in this embodiment, R₂ is hydrogen and R₃ is lower alkyl or ZY; Z is O, NR₄ or PR₄; Y is hydrogen or lower alkyl; ZY is NR₄NR₅R₇, NR₄OR₅, 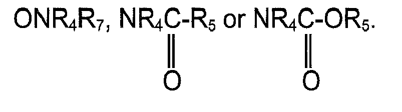 In another especially preferred embodiment, n=1, R₂ is hydrogen and R₃ is lower alkyl which may be substituted or unsubstituted with an electron donating or electron withdrawing group, NR₄OR₅, or ONR₄R₇,

In yet another especially preferred embodiment, n = 1, R₂ is hydrogen and R₃ is lower alkyl which is unsubstituted or substituted with hydroxy or loweralkoxy, NR₄OR₅ or ONR₄R₇, wherein R₄, R₅ and R₇ are independently hydrogen or lower alkyl, R is aryl lower alkyl, which aryl group may be unsubstituted or substituted with an electron withdrawing group and R₁ is lower alkyl. In this embodiment it is most preferred that aryl is phenyl, which is unsubstituted or substituted with halo.

It is preferred that R₂ is hydrogen and R₃ is hydrogen, an alkyl group which is unsubstituted or substituted by at least an electron donating or electron withdrawing group or ZY. In this preferred embodiment, it is more preferred that R₃ is hydrogen, an alkyl group such as methyl, which is unsubstituted or substituted by an electron donating group, or NR₄OR₅ or ONR₄R₇, wherein R₄, R₅ and R₇ are independently hydrogen or lower alkyl. It is preferred that the electron donating group is lower alkoxy, and especially methoxy or ethoxy.

It is preferred that R₂ and R₃ are independently hydrogen, lower alkyl, or ZY; Z is O, NR₄ or PR₄;
Y is hydrogen or lower alkyl or
ZY is NR₄R₅R₇, NR₄OR₅, ONR₄R₇,

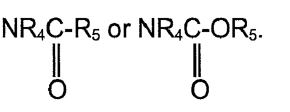

It is also preferred that R is aryl lower alkyl. The most preferred aryl for R is phenyl. The most preferred R group is benzyl. In a preferred embodiment, the aryl group may be unsubstituted or substituted with an electron donating or electron withdrawing group. If the aryl ring in R is substituted, it is most preferred that it is substituted with an electron withdrawing group, especially on the aryl ring. The most preferred electron withdrawing group for R is halo, especially fluoro.

The preferred R₁ is lower alkyl, especially methyl.

It is more preferred that R is aryl lower alkyl and R₁ is lower alkyl.

Further preferred compounds are compounds of Formula (Ib) wherein n is 1; R₂ is hydrogen; R₃ is hydrogen, a lower alkyl group, especially methyl which is substituted by an electron donating or electron withdrawing group or ZY; R is aryl, aryl lower alkyl, such as benzyl, wherein the aryl group is unsubstituted or substituted with an electron donating or electron withdrawing group and R₁ is lower alkyl. In this embodiment, it is more preferred that R₃ is hydrogen, a lower alkyl group, especially methyl, which may be substituted by electron donating group, such as lower alkoxy, (e.g., methoxy, ethoxy and the like), NR₄OR₅ or ONR₄R₇ wherein these groups are defined hereinabove.

The most preferred compounds utilized are those of the Formula (IIb): 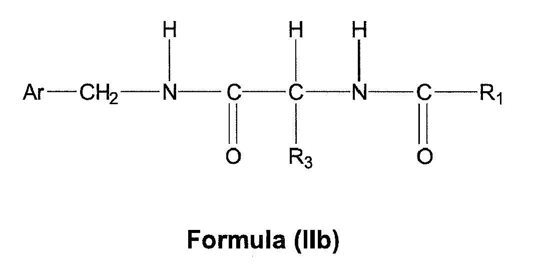 wherein
Ar is aryl, especially phenyl, which is unsubstituted or substituted with at least one electron donating group or electron withdrawing group, especially halo,
R₁ is lower alkyl, especially containing 1-3 carbon atoms; and
R₃ is as defined herein, but especially hydrogen, lower alkyl, which is unsubstituted or substituted by at least an electron donating group or electron withdrawing group or ZY. It is even more preferred that R₃ is, in this embodiment, hydrogen, an alkyl group which is unsubstituted or substituted by an electron donating group, NR₄OR₅ or ONR₄R₇. It is most preferred that R₃ is CH₂-Q, wherein Q is lower alkoxy, especially containing 1-3 carbon atoms; NR₄OR₅ or ONR₄R₇ wherein R₄ is hydrogen or alkyl containing 1-3 carbon atoms, R₅ is hydrogen or alkyl containing 1-3 carbon atoms, and R₇ is hydrogen or alkyl containing 1-3 carbon atoms.

The most preferred R₁ is CH₃. The most preferred R₃ is CH₂-Q, wherein Q is methoxy.

The most preferred aryl is phenyl. The most preferred halo is fluoro.

Yet another preferred embodiment refers to n=1 and the combination of R₁ being lower alkyl containing 1-6 carbon atoms, preferably 1-3 carbon atoms, more preferably methyl; R₂ being H and R in Formula (Ib) being unsubstituted or substituted benzyl, in particular halosubstituted benzyl, or Ar in Formula (IIb) being unsubstituted or substituted phenyl, in particular halosubstituted phenyl. In this combination, R₃ is as defined herein.

In another aspect, the compound of the present invention, in particular the compound of Formula (Ib), is represented by Formula (III) 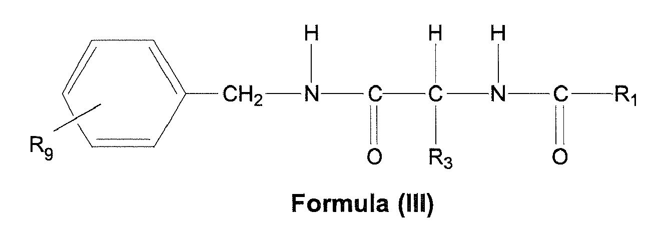 or a pharmaceutically acceptable salt thereof, wherein
- R₉: is one or more substituents independently selected from the group consisting of hydrogen, halo, alkyl, alkenyl, alkynyl, nitro, carboxy, formyl, carboxyamido, aryl, quaternary ammonium, haloalkyl, aryl alkanoyl, hydroxy, alkoxy, amino, alkylamino, dialkylamino, aryloxy, mercapto, alkylthio, alkylmercapto, and disulfide;
- R₃: is selected from the group consisting of hydrogen, alkyl, alkoxy, alkoxyalkyl, aryl, N-alkoxy-N-alkylamino, and N-alkoxyamino; and
- R₁: is alkyl.

Alkyl, alkoxy, alkenyl and alkynyl groups in a compound of Formula (III) are lower alkyl, alkoxy, alkenyl and alkynyl groups having no more than 6, more typically no more than 3, carbon atoms.

In a particular aspect, R₉ substituents in a compound of Formula (III) are independently selected from hydrogen and halo, more particularly fluoro, substituents.

In a particular aspect, R₃ in a compound of Formula (III) is alkoxyalkyl, phenyl, N-alkoxy-N-alkylamino or N-alkoxyamino.

In a particular aspect, R₁ in a compound of Formula (III) is C₁₋₃ alkyl.

In a more particular aspect, no more than one R₉ substituent is fluoro and all others are hydrogen; R₃ is selected from the group consisting of methoxymethyl, phenyl, N-methoxy-N-methylamino and N-methoxyamino; and R₁ is methyl.

The most preferred compounds include:
(R)-2-acetamido-N-benzyl-3-methoxy-propionamide (lacosamide);
(R)-2-acetamido-N-benzyl-3-ethoxy-propionamide;
O-methyl-N-acetyl-D-serine-m-fluorobenzyl-amide;
O-methyl-N-acetyl-D-serine-p-fluorobenzyl-amide;
N-acetyl-D-phenylglycine benzylamide;
D-1,2-(N,O-dimethylhydroxylamino)-2-acetamide acetic acid benzylamide;
D-1,2-(O-methylhydroxylamino)-2-acetamido acetic acid benzylamide.

It is to be understood that the various combinations and permutations of the Markush groups of R₁, R₂, R₃, R and n are contemplated to be within the scope of the present invention. Moreover, the present invention also encompasses compounds and compositions which contain one or more elements of each of the Markush groupings in R₁, R₂, R₃, n and R and the various combinations thereof. Thus, for example, the present invention contemplates that R₁ may be one or more of the substituents listed hereinabove in combination with any and all of the substituents of R₂, R₃, and R with respect to each value of n.

The compounds utilized in the present invention may contain one or more asymmetric carbons and may exist in racemic and optically active forms. The configuration around each asymmetric carbon can be either the D or L form. It is well known in the art that the configuration around a chiral carbon atoms can also be described as R or S in the Cahn-Prelog-Ingold nomenclature system. All of the various configurations around each asymmetric carbon, including the various enantiomers and diastereomers as well as racemic mixtures and mixtures of enantiomers, diastereomers or both are contemplated by the present invention.

In the principal chain, there exists asymmetry at the carbon atom to which the groups R₂ and R₃ are attached. When n is 1, the compounds of the present invention is of the formula 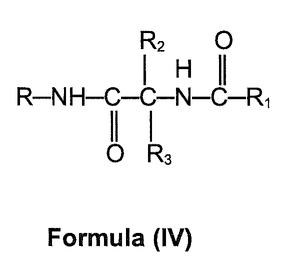 wherein R, R₁, R₂, R₃, R₄, R₅, R₆, R'₆, R₇, R₈, R₅₀, Z and Y are as defined previously.

As used herein, the term configuration shall refer to the configuration around the carbon atom to which R₂ and R₃ are attached, even though other chiral centers may be present in the molecule. Therefore, when referring to a particular configuration, such as D or L, it is to be understood to mean the D or L stereoisomer at the carbon atom to which R₂ and R₃ are attached. However, it also includes all possible enantiomers and diastereomers at other chiral centers, if any, present in the compound.

The compounds of the present invention are directed to all the optical isomers, i.e., the compounds of the present invention are either the L-stereoisomer or the D-stereoisomer (at the carbon atom to which R₂ and R₃ are attached). These stereoisomers may be found in mixtures of the L and D stereoisomer, e.g., racemic mixtures. The D stereoisomer is preferred.

More preferred is a compound of Formula (IV) in the R configuration, preferably substantially enantiopure, wherein the substituent R is benzyl which is unsubstituted with at least one halo group, wherein R₃ is CH₂-Q, wherein Q is lower alkoxy containing 1-3 carbon atoms and wherein R₁ is methyl. Preferably R is unsubstituted benzyl or benzyl substituted with at least one halo group which is a fluoro group.

A "substantially enantiopure" compound of the present invention may have at least 88%, preferably at 90%, more preferably at least 95, 96, 97, 98, or 99% enantiomeric purity.

Depending upon the substituents, the present compounds may form addition salts as well. All of these forms are contemplated to be within the scope of this invention including mixtures of the stereoisomeric forms.

The manufacture of the utilized compounds is described in U.S. Patent Nos. 5,378,729 and 5,773.475, the contents of both of which are incorporated by reference.

The compounds utilized in the present invention are useful as such as depicted in the Formulae (Ib) or/and (IIb) or can be employed in the form of salts in view of its basic nature by the presence of the free amino group. Thus, the compounds of Formulae (Ib) or/and (IIb) form salts with a wide variety of acids, inorganic and organic, including pharmaceutically acceptable acids. The salts with therapeutically acceptable acids are of course useful in the preparation of formulation where enhanced water solubility is most advantageous.

These pharmaceutically acceptable salts have also therapeutic efficacy. These salts include salts of inorganic acids such as hydrochloric, hydroiodic, hydrobromic, phosphoric, metaphosphoric, nitric acid and sulfuric acids as well as salts of organic acids, such as tartaric, acetic, citric, malic, benzoic, perchloric, glycolic, gluconic, succinic, aryl sulfonic, (e.g., p-toluene sulfonic acids, benzenesulfonic), phosphoric, malonic, and the like.

The present invention is further directed to a method for the prevention, alleviation or/and treatment of a disease or condition as described above in a mammal, including a human being, comprising administering at least one compound of formulae (Ib) or/and (IIb).

It is preferred that the compound utilized in the present invention is used in therapeutically effective amounts.

The physician will determine the dosage of the present therapeutic agents which will be most suitable and it will vary with the form of administration and the particular compound chosen, and furthermore, it will vary with the patient under treatment, the age of the patient, the type of malady being treated. He will generally wish to initiate treatment with small dosages substantially less than the optimum dose of the compound and increase the dosage by small increments until the optimum effect under the circumstances is reached. When the composition is administered orally, larger quantities of the active agent will be required to produce the same effect as a smaller quantity given parenterally. The compounds are useful in the same manner as comparable therapeutic agents and the dosage level is of the same order of magnitude as is generally employed with these other therapeutic agents.

In a preferred embodiment, the compounds of the present invention are administered in amounts ranging from about 1 mg to about 100 mg per kilogram of body weight per day, more preferably in amounts ranging from about 1 mg to about 10 mg per kilogram of body weight per day. This dosage regimen may be adjusted by the physician to provide the optimum therapeutic response. Patients in need thereof may be treated with doses of the compound of the present invention of at least 50 mg/day, preferably of at least 200 mg/day, more preferably of at least 300 mg/day and most preferably of at least 400 mg/day. Generally, a patient in need thereof may be treated with doses at a maximum of 6 g/day, more preferably a maximum of 1 g/day and most preferably a maximum of 600 mg/day. In some cases, however, higher or lower doses may be needed.

In another preferred embodiment, the daily doses are increased until a predetermined daily dose is reached which is maintained during the further treatment.

In yet another preferred embodiment, several divided doses may be administered daily. For example, three doses per day may be administered, preferably two doses per day. It is more preferred to administer a single dose per day.

In yet another preferred embodiment, an amount of the compounds of the present invention may be administered which results in a plasma concentration of 0.1 to 15 µg/ml (trough) and 5 to 18.5 µg/ml (peak), calculated as an average over a plurality of treated subjects.

The compounds of Formulae (Ib) or/and (IIb) may be administered in a convenient manner, such as by oral, intravenous (where water soluble), intramuscular, intrathecal or subcutaneous routes. Oral or/and i.v. administration is preferred.

The pharmaceutical composition of the present invention may be prepared for the treatment regimen as described above, in particular for the treatment with doses as described above, to effect plasma concentrations as described above, for administration periods or/and administration routes as specified in the embodiments of the present invention as described above.

In another preferred embodiment, the method of the present invention as described above for the treatment of a mammal including a human being in need thereof comprises administering a compound of the present invention in combination with administering a further active agent for the prevention, alleviation or/and treatment, in particular systemic treatment of non-inflammatory musculoskeletal pain or/and non-inflammatory osteoarthritic pain, in particular of specific manifestations of non-inflammatory musculoskeletal pain or/and non-inflammatory osteoarthritic pain, such as muscular hyperalgesia or/and allodynia occurring in fibromyalgia, myofascial pain syndrome back pain or/and osteoarthritis. The compound of the present invention and the further active agent may be administered together, i.e. in a single dose form, or may be administered separately, i.e. in a separate dose form. Thus, the pharmaceutical composition of the present invention may comprise a compound of the present invention as defined above and may further comprise a further active agent for the prevention, alleviation or/and treatment, in particular systemic treatment of non-inflammatory musculoskeletal pain or/and non-inflammatory osteoarthritic pain, in particular specific manifestations of non-inflammatory musculoskeletal pain or/and non-inflammatory osteoarthritic pain, such as muscular hyperalgesia or/and allodynia occurring in fibromyalgia, myofascial pain syndrome, back pain or/and osteoarthritis. The pharmaceutical composition may comprise a single dose form or may comprise a separate dose form comprising a first composition comprising a compound of the present invention as defined above and a second composition comprising the further active agent.

In yet another preferred embodiment, the method of the present invention comprises administering a compound of the present invention in combination with administering a further active agent for the prevention, alleviation or/and treatment of non-inflammatory arthritic pain. The pharmaceutical composition of the present invention may comprise a compound of the present invention as defined above and may comprise a further active agent for the prevention, alleviation or/and treatment of non-inflammatory arthritic pain, such as pain associated with or/and caused by osteoarthritis.

The two or more active agents of the above combinations can be formulated in one pharmaceutical preparation (single dosage form) for administration to the subject at the same time, or in two or more distinct preparations (separate dosage forms) for administration to the subject substantially at the same time or at different times or frequencies, *e.g*., sequentially. The two distinct preparations can be formulated for administration by the same route or by different routes.

Separate dosage forms can optionally be co-packaged, for example in a single container or in a plurality of containers within a single outer package, or co-presented in separate packaging ("common presentation"). As an example of co-packaging or common presentation, a kit is contemplated comprising, in a first container, the compound of Formulas (Ib), (IIb) or (III) and, in a second container, the second active agent or the at least one anti-osteoarthritis agent. In another example, the compound of Formulas (Ib), (IIb) or (III) and the second active agent or the at least one anti-osteoarthritis agent are separately packaged and available for sale independently of one another, but are co-marketed or copromoted for use according to the invention. The separate dosage forms may also be presented to a subject separately and independently, for use according to the invention.

Depending on the dosage forms, which may be identical or different, *e.g.*, fast release dosage forms, controlled release dosage forms or depot forms, the compound of Formulas (Ib), (IIb) or (III) and the second active agent or the anti-osteoarthritis agent may be administered on the same or on different schedules, for example on a daily, weekly or monthly basis.

The term "therapeutic combination" refers to a plurality of agents that, when administered to a subject together or separately, are co-active in bringing therapeutic benefit to the subject. Such administration is referred to as "combination therapy," "cotherapy," "adjunctive therapy" or "add-on therapy." For example, one agent can potentiate or enhance the therapeutic effect of another, or reduce an adverse side effect of another, or one or more agents can be effectively administered at a lower dose than when used alone, or can provide greater therapeutic benefit than when used alone, or can complementarily address different aspects, symptoms or etiological factors of a disease or condition.

The further active agent for the prevention, alleviation or/and treatment of non-inflammatory musculoskeletal pain or/and non-inflammatory osteoarthritic pain, in particular of the specific manifestation of non-inflammatory musculoskeletal pain or/and non-inflammatory osteoarthritic pain may be a compound different from that of Formulae (Ib) or/and (IIb), in particular an anticonvulsant, which anticonvulsant is preferably selected from first generation anticonvulsants, such as carbamazepine and phenytoin, and second generation anticonvulsants, such as gabapentin, pregabalin, lamotrigine, and levetiracetam.

The further active agent for the prevention, alleviation or/and treatment of non-inflammatory musculoskeletal pain or/and non-inflammatory osteoarthritic pain as described herein may also be employed as further active agent for the prevention, alleviation or/and treatment of non-inflammatory arthritic pain.

The compounds of the present invention may be used for the preparation of a pharmaceutical composition as described above.

The further active agent for the prevention, alleviation or/and treatment of non-inflammatory musculoskeletal pain or/and non-inflammatory osteoarthritic pain can be at least one anti-osteoarthritic agent, i. e. the further active agent may also be effective for prevention, alleviation or/and treatment of osteoarthritis or any aspect, symptom or underlying cause thereof. In the present invention, the at least one anti-osteoarthritic agent may be an agent other than a compound of Formulae (Ib) or/and (IIb), in particular other than an anticonvulsant.

In one embodiment, the further active agent for the prevention, alleviation or/and treatment of non-inflammatory musculoskeletal pain or/and non-inflammatory osteoarthritic pain, in particular the anti-osteoarthritis agent is effective for treatment of pain, *i.e*., analgesia. Suitable analgesics include opioid and non-opioid analgesics as well as certain anti-inflammatory drugs (see immediately below).

As indicated herein, osteoarthritis pain can comprise both a non-inflammatory and inflammatory component. Therefore, in another embodiment the further active agent, in particular the anti-osteoarthritis agent is effective for treating inflammation and/or pain related thereto. Suitable antiinflammatories include steroidal and nonsteroidal anti-inflammatory drugs. Nonsteroidal anti-inflammatory drugs (NSAIDs) include traditional NSAIDs and cyclooxygenase-2 (COX-2) selective inhibitors.

Nonlimiting examples of opioid and non-opioid analgesics that can be useful as the further active agent, in particular as the anti-osteoarthritis agent for administration in combination or adjunctive therapy with a compound of Formulas (Ib), (IIb) or (III), *e.g.*, lacosamide, include acetaminophen, alfentanil, allylprodine, alphaprodine, anileridine, benzylmorphine, bezitramide, buprenorphine, butorphanol, clonitazene, codeine, cyclazocine, desomorphine, dextromoramide, dextropropoxyphene, dezocine, diampromide, diamorphone, dihydrocodeine, dihydromorphine, dimenoxadol, dimepheptanol, dimethylthiambutene, dioxaphetyl butyrate, dipipanone, eptazocine, ethoheptazine, ethylmethylthiambutene, ethylmorphine, etonitazene, fentanyl, heroin, hydrocodone, hydromorphone, hydroxypethidine, isomethadone, ketobemidone, levallorphan, levorphanol, levophenacylmorphan, lofentanil, meperidine, meptazinol, metamizol, metazocine, methadone, metopon, morphine, myrophine, nalbuphine, nalorphine, narceine, nicomorphine, norlevorphanol, normethadone, normorphine, norpipanone, opium, oxycodone, oxymorphone, papaveretum, pentazocine, phenadoxone, phenazocine, phenomorphan, phenoperidine, piminodine, piritramide, proheptazine, promedol, properidine, propiram, propoxyphene, sufentanil, tilidine, tramadol, NO-naproxen, NCX-701, ALGRX-4975, and combinations thereof.

Nonlimiting examples of steroidal anti-inflammatories that can be useful as the further active agent, in particular as the anti-osteoarthritis agent for administration in combination or adjunctive therapy with a compound of Formulas (Ib), (IIb) or (III), *e.g.*, lacosamide, include alclometasone, amcinonide, betamethasone, betamethasone 17-valerate, clobetasol, clobetasol propionate, clocortolone, cortisone, dehydrotestosterone, deoxycorticosterone, desonide, desoximetasone, dexamethasone, dexamethasone 21-isonicotinate, diflorasone, fluocinonide, fluocinolone, fluorometholone, flurandrenolide, fluticasone, halcinonide, halobetasol, hydrocortisone, hydrocortisone acetate, hydrocortisone cypionate, hydrocortisone hemisuccinate, hydrocortisone 21-lysinate, hydrocortisone sodium succinate, isoflupredone, isoflupredone acetate, methylprednisolone, methylprednisolone acetate, methylprednisolone sodium succinate, methylprednisolone suleptanate, mometasone, prednicarbate, prednisolone, prednisolone acetate, prednisolone hemisuccinate, prednisolone sodium phosphate, prednisolone sodium succinate, prednisolone valerate-acetate, prednisone, triamcinolone, triamcinolone acetonide and combinations thereof.

Nonlimiting examples of NSAIDs and COX-2 selective inhibitors that can be useful as the further active agent, in particular as the anti-osteoarthritis agent for administration in combination or adjunctive therapy with a compound of Formulas (Ib), (IIb) or (III), *e.g.*, lacosamide, include salicylic acid derivatives (such as salicylic acid, acetylsalicylic acid, methyl salicylate, diflunisal, olsalazine, salsalate and sulfasalazine), indole and indene acetic acids (such as indomethacin, etodolac and sulindac), fenamates (such as etofenamic, meclofenamic, mefenamic, flufenamic, niflumic and tolfenamic acids), heteroaryl acetic acids (such as acemetacin, alclofenac, clidanac, diclofenac, fenchlofenac, fentiazac, furofenac, ibufenac, isoxepac, ketorolac, oxipinac, tiopinac, tolmetin, zidometacin and zomepirac), aryl acetic acid and propionic acid derivatives (such as alminoprofen, benoxaprofen, bucloxic acid, carprofen, fenbufen, fenoprofen, fluprofen, flurbiprofen, ibuprofen, indoprofen, ketoprofen, miroprofen, naproxen, naproxen sodium, oxaprozin, pirprofen, pranoprofen, suprofen, tiaprofenic acid and tioxaprofen), enolic acids (such as the oxicam derivatives ampiroxicam, cinnoxicam, droxicam, lornoxicam, meloxicam, piroxicam, sudoxicam and tenoxicam, and the pyrazolone derivatives aminopyrine, antipyrine, apazone, dipyrone, oxyphenbutazone and phenylbutazone), alkanones (such as nabumetone), nimesulide, proquazone, MX-1094, licofelone, and combinations thereof.

In yet another embodiment the further active agent, in particular the anti-osteoarthritic agent, is an anti-inflammatory agent of the class of COX-2 selective inhibitors, selected for example from celecoxib, deracoxib, valdecoxib, parecoxib, rofecoxib, etoricoxib, lumiracoxib, 2-(3,5-difluorophenyl)-3-[4- (methylsulfonyl)phenyl]-2-cyclopenten-1-one, (S)-6,8-dichloro-2-(trifluoromethyl)-2H-1- benzopyran-3-carboxylic acid, 2-(3,4-difluorophenyl)-4-(3-hydroxy-3-methyl-1-butoxy)-5- [4-(methylsulfonyl)phenyl]-3-(2H)-pyridazinone, 4-[5-(4-fluorophenyl)-3-(trifluoromethyl)- 1H-pyrazol-1-yl]benzenesulfonamide, 4-[5-(phenyl)-3-(trifluoromethyl)-1H-pyrazol-1-yl]benzenesulfonamide, PAC-10549, cimicoxib, GW-406381, LAS-34475, CS-502 and combinations thereof.

In another embodiment, the further active agent for the prevention, alleviation or/and treatment of non-inflammatory musculoskeletal pain or/and non-inflammatory osteoarthritic pain, in particular the anti-osteoarthritis agent is at least one antidepressant. Such combination or adjunctive therapies can, in some situations, be more effective in treatment of non-inflammatory musculoskeletal pain or/and non-inflammatory osteoarthritic pain, and/or can have reduced adverse side effects than monotherapies with the compound of Formulas (Ib), (IIb) or (III), for example lacosamide, or the antidepressant alone.

Nonlimiting examples of antidepressants that can be useful in combination or adjunctive therapy with a compound of Formulas (Ib), (IIb) or (III), *e.g*., lacosamide, include without limitation bicyclic, tricyclic and tetracyclic antidepressants, hydrazides, hydrazines, phenyloxazolidinones and pyrrolidones. Specific examples include adinazolam, adrafinil, amineptine, amitriptyline, amitriptylinoxide, amoxapine, befloxatone, bupropion, butacetin, butriptyline, caroxazone, citalopram, clomipramine, cotinine, demexiptiline, desipramine, dibenzepin, dimetacrine, dimethazan, dioxadrol, dothiepin, doxepin, duloxetine, etoperidone, femoxetine, fencamine, fenpentadiol, fluacizine, fluoxetine, fluvoxamine, hematoporphyrin, hypericin, imipramine, imipramine N-oxide, indalpine, indeloxazine, iprindole, iproclozide, iproniazid, isocarboxazid, levophacetoperane, lofepramine, maprotiline, medifoxamine, melitracen, metapramine, metralindole, mianserin, milnacipran, minaprine, mirtazapine, moclobemide, nefazodone, nefopam, nialamide, nomifensine, nortriptyline, noxiptilin, octamoxin, opipramol, oxaflozane, oxitriptan, oxypertine, paroxetine, phenelzine, piberaline, pizotyline, prolintane, propizepine, protriptyline, pyrisuccideanol, quinupramine, reboxetine, ritanserin, roxindole, rubidium chloride, sertraline, sulpiride, tandospirone, thiazesim, thozalinone, tianeptine, tofenacin, toloxatone, tranylcypromine, trazodone, trimipramine, tryptophan, venlafaxine, viloxazine, zimeldine, and combinations thereof.

In another embodiment, the further active agent for the prevention, alleviation or/and treatment of non-inflammatory musculoskeletal pain or/and non-inflammatory osteoarthritic pain, in particular the anti-osteoarthritis agent is at least one NMDA receptor antagonist. Such combination or adjunctive therapies can, in some situations, be more effective in treatment of non-inflammatory musculoskeletal pain or/and non-inflammatory osteoarthritic pain, and/or can have reduced adverse side effects than monotherapies with the compound of Formulas (Ib), (IIb) or (III), for example lacosamide, or the NMDA receptor antagonist alone.

Nonlimiting examples of NMDA receptor antagonists that can be useful in combination or adjunctive therapy with a compound of Formulas (Ib), (IIb) or (III), *e.g.*, lacosamide, include aptiganel, dexanabinol, licostinel, memantine, remacemide, and combinations thereof.

In yet another embodiment, the further active agent, in particular the anti-osteoarthritis agent, is a disease-modifying osteoarthritis drug (DMOAD). The term "DMOAD" herein refers to any drug that has utility in treatment of osteoarthritis or symptoms thereof, other than those mentioned above. Nonlimiting examples of DMOADs that can be useful as the further active agent, in particular as the anti-osteoarthritis agent for administration in combination or adjunctive therapy with a compound of Formulas (Ib), (IIb) or (III), *e.g.*, lacosamide, include methotrexate, diacerein, glucosamine, chondroitin sulfate, anakinra, MMP inhibitors, doxycycline, minocycline, misoprostol, proton pump inhibitors, non-acetylated salicylates, tamoxifen, prednisone, methylprednisolone, polysulfated glycosaminoglycan, calcitonin, alendronate, risedronate, zoledronic acid, teriparatide, VX-765, pralnacasan, SB-462795, CPA-926, ONO-4817, S-3536, PG-530742, CP-544439, and combinations thereof.

In yet another embodiment, the further active agent, in particular the anti-osteoarthritis agent, is a symptom modifying osteoarthritis drug other than those mentioned above. Nonlimiting examples of such drugs that can be useful as the further active agent, in particular as the anti-osteoarthritis agent for administration in combination or adjunctive therapy with a compound of Formulas (Ib), (IIb) or (III), *e.g.*, lacosamide, include ADL-100116, AD-827, HOE-140, DA-5018, and combinations thereof.

Suitable regimens including doses and routes of administration for particular anti-osteoarthritis agents can be determined from readily-available reference sources relating to these agents, for example Physicians' Desk Reference (PDR), 60th edition, Montvale, NJ: Thomson (2006) and various internet sources known to those of skill in the art. When administered in combination or adjunctive therapy with a compound of Formulas (Ib), (IIb) or (III), for example lacosamide, the anti-osteoarthritis agent can be used at a full dose, but the physician may elect to administer less than a full dose of the antiosteoarthritis agent, at least initially.

More than one anti-osteoarthritis agent can be administered in combination or adjunctive therapy with a compound of Formulas (Ib), (IIb) or (III), for example lacosamide. In one embodiment two or more such agents are included in the combination or adjunctive therapy, selected from two or more of the following classes:
(i) opioid and non-opioid analgesics;
(ii) steroidal anti-inflammatories;
(iii) NSAIDs and COX-2 selective inhibitors; and
(iv) DMOADs.

The compounds of Formulae (Ib) or/and (IIb) may be orally administered, for example, with an inert diluent or with an assimilable edible carrier, or it may be enclosed in hard or soft shell gelatin capsules, or it may be compressed into tablets, or it may be incorporated directly into the fool of the diet. For oral therapeutic administration, the active compound of Formulae (Ib) or/and (IIb) may be incorporated with excipients and used in the form of ingestible tablets, buccal tablets, troches, capsules, elixirs, suspensions, syrups, wafers, and the like. Such compositions and preparations should contain at least 1 % of active compound of Formulae (Ib) or/and (IIb). The percentage of the compositions and preparations may, of course, be varied and may conveniently be between about 5 to about 80 % of the weight of the unit. The amount of active compound of Formulae (Ib) or/and (IIb) in such therapeutically useful compositions is such that a suitable dosage will be obtained. Preferred compositions or preparations according to the present invention contains between about 10 mg and 6 g active compound of Formulae (Ib) or/and (IIb).

The tablets, troches, pills, capsules and the like may also contain the following: A binder such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, lactose or saccharin may be added or a flavoring agent such as peppermint, oil of wintergreen, or cherry flavoring. When the dosage unit form is a capsule, it may contain, in addition to materials of the above type, a liquid carrier.

Various other materials may be present as coatings or otherwise modify the physical form of the dosage unit. For instance, tablets, pills, or capsules may be coated with shellac, sugar or both. A syrup or elixir may contain the active compound, sucrose as a sweetening agent, methyl and propylparabens as preservatives, a dye and flavoring such as cherry or orange flavor. Of course, any material used in preparing any dosage unit form should be pharmaceutically pure and substantially non-toxic in the amounts employed. In addition, the active compound may be incorporated into sustained-release preparations and formulations. For example, sustained release dosage forms are contemplated wherein the active ingredient is bound to an ion exchange resin which, optionally, can be coated with a diffusion barrier coating to modify the release properties of the resin.

The active compound may also be administered parenterally or intraperitoneally. Dispersions can also be prepared in glycerol, liquid, polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions (where water soluble) or dispersions and sterile powders for the extemporaneous preparation of sterile injectable solutions or dispersions. In all cases the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating such as lecithin, by the maintenance of the required particle size in the case of dispersions and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active compound in the required amount in the appropriate solvent with various of the other ingredients enumerated above, as required, followed by filtered sterilization. Generally, dispersions are prepared by incorporating the various sterilized active ingredient into a sterile vehicle which contains the basic dispersion medium and the required other ingredients from those enumerated above. In the case of sterile powders for the preparation of sterile injectable solutions, the preferred methods of preparation are vacuum drying the freeze-drying technique plus any additional desired ingredient from previously sterile-filtered solution thereof.

As used herein, "pharmaceutically acceptable carrier" includes any and all solvents, dispersion media, coatings, antibacterial and antifungal agent, isotonic and absorption delaying agents for pharmaceutical active substances as well known in the art. Except insofar as any conventional media or agent is incompatible with the active ingredient, its use in the therapeutic compositions is contemplated. Supplementary active ingredients can also be incorporated into the compositions.

It is especially advantageous to formulate parenteral compositions in dosage unit form or ease of administration and uniformity of dosage. Dosage unit form as used herein refers to physically discrete units suited as unitary dosages for the mammalian subjects to be treated; each unit containing a predetermined quantity of active material calculated to produce the desired therapeutic effect in association with the required pharmaceutical carrier. The specifics for the novel dosage unit forms of the invention are dictated by and directly dependent on (a) the unique characteristics of the active material an the particular therapeutic effect to be achieved, and (b) the limitations inherent in the art of compounding such as active material for the treatment of disease in living subjects having a diseased condition in which bodily health is impaired as herein disclosed in detail.

The principal active ingredient is compounded for convenient and effective administration in effective amounts with a suitable pharmaceutically acceptable carrier in dosage unit form as hereinbefore described. A unit dosage form can, for example, contain the principal active compound in amounts ranging from about 10 mg to about 6 g. Expressed in proportions, the active compound is generally present in from about 1 to about 750 mg/ml of carrier. In the case of compositions containing supplementary active ingredients, the dosages are determined by reference to the usual dose and manner of administration of the said ingredients.

As used herein the term "patient" or "subject" refers to a warm blooded animal, and preferably mammals, such as, for example, cats, dogs, horses, cows, pigs, mice, rats and primates, including humans. The preferred patient is a human, for example a patient having clinically diagnosed osteoarthritis, fibromyalgia, or myofascial pain syndrome.

The term "treat" refers to either relieving the pain associated with a disease or condition, to curing or alleviating the patient's disease or condition.

The compounds of the present invention are administered to a patient suffering from the aforementioned type of disorder in an effective amount. These amounts are equivalent to the therapeutically effective amounts described hereinabove.

The following Example 1 shows the ability of lacosamide to inhibit mechanical hyperalgesia after musculoskeletal pain induced by TNF in the rat. Additionally, lacosamide attenuates mechanical allodynia in the same model measured by grip strength. The model reflects musculoskeletal pain which occurs in fibromyalgia, myofascial pain syndrome or back pain.

The following Example 2 shows that lacosamide inhibited mechanical hyperalgesia during the post-inflammatory period in the iodoacetate rat model of osteoarthritic pain, indicating effectiveness of lacosamide for treating noninflammatory osteoarthritic pain.

The substance used in Example 1 and 2 was lacosamide (international non-proprietary name) which is the synonym for Harkoseride or SPM 927. The standard chemical nomenclature is (R)-2-acetamide-N-benzyl-3-methoxypropionamide.

### Figure legends

**Figure 1** shows the effect of lacosamide at 3 mg/kg, 10 mg/kg, and 30 mg/kg on paw withdrawal after muscle pressure after TNF-induced muscle pain.
**Figure 2** shows the effect of lacosamide at 3 mg/kg, 10 mg/kg, and 30 mg/kg on paw withdrawal after muscle pressure after TNF-induced muscle pain in comparison to pregabalin, gabapentin, and metamizol. MPE: maximal possible effect.
**Figure 3** shows the effect of lacosamide at 3 mg/kg, 10 mg/kg, and 30 mg/kg on grip strength after TNF-induced muscle pain.
**Figure 4** shows the effect of lacosamide at 3 mg/kg, 10 mg/kg, and 30 mg/kg on grip strength after TNF-induced muscle pain in comparison to pregabalin, gabapentin, and metamizol. MPE: maximal possible effect.
**Figs. 5A-C** are graphical representations of results obtained in the iodoacetate rat model of Example 2 after administration of lacosamide and morphine on tactile allodynia at days 3, 7 and 14 of the study respectively.
**Figs. 6A-C** are graphical representations of results obtained in the iodoacetate rat model of Example 2 after administration of diclofenac on tactile allodynia at days 3, 7 and 14 of the study respectively.
**Figs. 7A-C** are graphical representations of results obtained in the iodoacetate rat model of Example 2 after administration of lacosamide and morphine on mechanical hyperalgesia at days 3, 7 and 14 of the study respectively.
**Figs. 8A-C** are graphical representations of results obtained in the iodoacetate rat model of Example 2 after administration of diclofenac on mechanical hyperalgesia at days 3, 7 and 14 of the study respectively.

### Example 1

Intramuscular injection of tumor necrosis factor-alpha (TNF) was used as a model of muscular mechanical hyperalgesia, which occurs in the human fibromyalgia, myofascial pain syndrome, back pain or osteoarthritis.

Intramuscular injection of TNF induces mechanical muscle hyperalgesia in rats. This can be quantified by measuring the withdrawal threshold to muscle pressure and the grip strength. TNF injections do not lead to morphological damage of the muscle (1).

Pain on palpation of muscles without morphological abnormalities is typical of the fibromyalgia, myofascial pain syndrome or back pain in humans (2). Thus, the model of intramuscular injection of TNF can be used as a model of muscle pain related to fibromyalgia, myofascial pain syndrome or back pain. In this model the antinociceptive action of the new anticonvulsant lacosamide (LCM) was tested. Control drugs were the non-opioid analgesic metamizol (comparison example) and the anticonvulsants pregabalin and gabapentin.

Mechanical withdrawal thresholds to muscle pressure were measured with an analgesimeter exerting pressure on the gastrocnemius muscle previously injected with TNF. Forelimb grip strength was measured with a digital grip force meter after TNF injection into the biceps brachii muscles.

### Animals, induction of muscle pain

Adult male Sprague Dawley rats with a body weight of 250 g to 300 g were used (supplier: Charles River Sulzfeld Germany). Animals were group-housed (3 animals per cage) and maintained in a room with controlled temperature (21-22°C) and a reversed light-dark cycle (12 h/12 h) with food and water available ad libitum. All experiments were approved by the Bavarian State animal experimentation committee and carried out in accordance with its regulations.

Recombinant rat tumor necrosis factor alpha (herein referred to as TNF) was obtained from R&D Systems, Minneapolis, MN, USA. TNF was diluted in 0,9% NaCl and used in a concentration of 1 µg in 50 µl. Injections were performed in short halothane narcosis with a 30 g needle bilaterally into the gastrocnemius or into the biceps brachii muscle. All rats were used to the behavioral tests before injections and baseline values were recorded over three test days.

### Behavioral readout: muscle pressure (Randall-Selitto)

Mechanical withdrawal thresholds to muscle pressure were measured with an analgesimeter (Ugo Basile, Comerio, Italy). The rat is allowed to crawl into a sock which helps the rat to relax. The hind limbs were positioned such that an increasing pressure could be applied onto the gastrocnemius muscle (maximum 250 g). The pressure needed to elicit withdrawal was recorded. Means of 3 trials for each hind limb were calculated (interstimulus interval of >30 sec). Only animals with a significant TNF effect were included for further analysis.

Rats were injected with TNF into the M. gastrocnemius at 2 pm. 18 hours later, rats were tested for pressure hyperalgesia pre- and post-application of the drugs. Rats were tested for pressure hyperalgesia 30 to 60 minutes after drug administration.

### Behavioral readout: grip strength

Grip strength of the forelimbs was tested with a digital grip force meter (DFIS series, Chatillon, Greensboro, NC, USA). The rat was positioned to grab the grid with the forelimbs and was gently pulled so that the grip strength could be recorded. Means of three trials were calculated. The effect of the TNF treatment was calculated for each animal separately and only animals with a significant TNF effect were included for further analysis.

Rats were injected with TNF into the M. biceps brachii at 8 am. 6 hours later, grip strength of the forelimbs was tested with a digital grip force meter. Drugs were applied, and grip strength was again tested after 30 to 60 minutes.

### Application Protocol

The rats, 10 per group, were treated with either 3, 10 or 30 mg/kg lacosamide or the vehicle intraperitoneally (i.p.). Injection volume of i.p. injections was 0.5 ml. A pilot study was performed to show that injection of 1 µg TNF i.m. into the gastrocnemius muscle is sufficient to induce pressure hyperalgesia.

**Table 1: Injection of TNF into the Gastrocnemius Muscles**

| **Group No** | **Substance** | **Dose** | **Number of rats** |
|---|---|---|---|
| 1.1 | TNF 1 µg, Lacosamide | 3 mg/kg i.p. | 8 |
| 1.2 | TNF 1 µg, Lacosamide | 10 mg/kg i.p. | 8 |
| 1.3 | TNF 1 µg, Lacosamide | 30 mg/kg i.p. | 7 |
| 1.4 | TNF 1 µg, Pregabalin | 30 mg/kg i.p. | 8 |
| 1.5 | TNF 1 µg, Pregabalin | 100 mg/kg i.p. | 10 |
| 1.6 | TNF 1 µg, Gabapentin | 100 mg/kg i.p. | 10 |
| 1.7 | TNF 1 µg, NaCl | i.p. | 10 |
| 1.8 | TNF 1 µg, Metamizol | 2 mg/kg i.p. | 9 |

**Table 2: Injection of TNF into the Biceps Brachii Muscles**

| **Group No** | **Substance** | **Dose** | **Number of rats** |
|---|---|---|---|
| 2.1 | TNF 1 µg, Lacosamide | 3 mg/kg i.p. | 4 |
| 2.2 | TNF 1 µg, Lacosamide | 10 mg/kg i.p. | 9 |
| 2.3 | TNF 1 µg, Lacosamide | 30 mg/kg i.p. | 10 |
| 2.4 | TNF 1 µg, Pregabalin | 30mg/kg i.p. | 10 |
| 2.5 | TNF 1 µg, Pregabalin | 100 mg/kg i.p. | 10 |
| 2.6 | TNF 1 µg, Gabapentin | 100 mg/kg i.p. | 10 |
| 2.7 | TNF 1 µg, NaCl | i.p. | 10 |
| 2.8 | TNF 1 µg, Metamizol | 2 mg/kg i.p. | 7 |

### Data Presentation and Statistics

Data are shown in graphs displaying means and SEMs. Pre- and posttreatment data were compared using ANOVA (Analysis Of Variance) and a Tukey post hoc test. Means of treatment groups were compared using a one-way ANOVA and Dunnett's post hoc Test. Maximal possible effects (MPE) were calculated for all types of treatment.

### Results

### Muscle Pressure Hyperalgesia

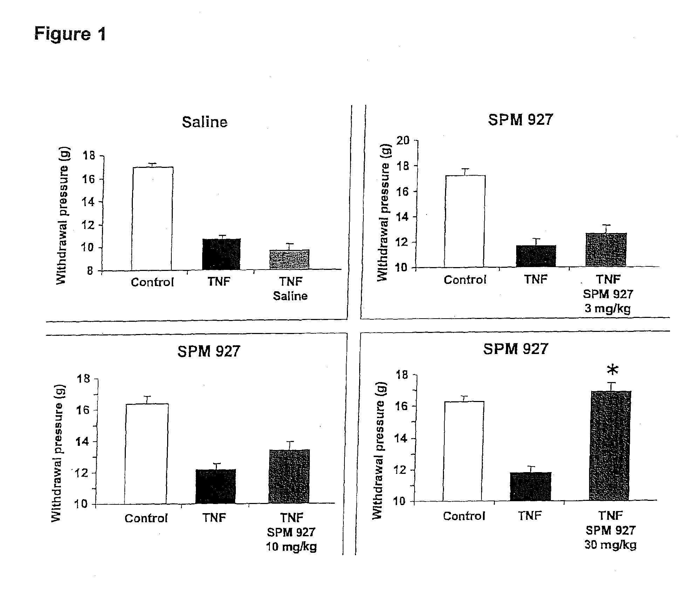

Only rats in which withdrawal thresholds were significantly reduced after TNF injection were included. In about 13% of the rats, the TNF effect was absent. Figure 1 shows the absolute values of withdrawal thresholds to pressure.

A complete reversal of muscular mechanical hyperalgesia was seen with lacosamide at 30 mg/kg and metamizol at 2 mg/kg.

A significant reversal of muscular mechanical hyperalgesia was also seen for pregabalin at 30 and 100 mg/kg, gabapentin at 100 mg/kg.

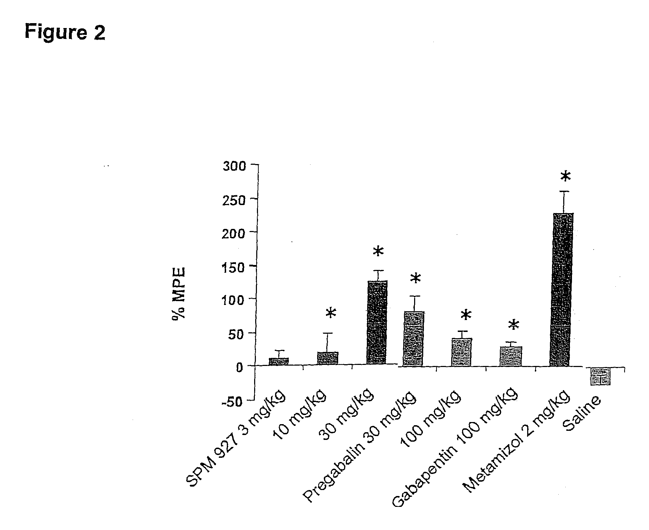

The Maximal Possible Effect (Figure 2) was significantly different from vehicle for lacosamide at 10 and 30 mg/kg, for pregabalin at 30 and 100 mg/kg, for gabapentin at 100 mg/kg, and for metamizol at 2 mg/kg. The vehicles had no effect.

### Biceps muscle grip strength

Only rats in which the grip strength was significantly reduced after TNF injection were included. In about 13% of the rats, the TNF effect was absent.

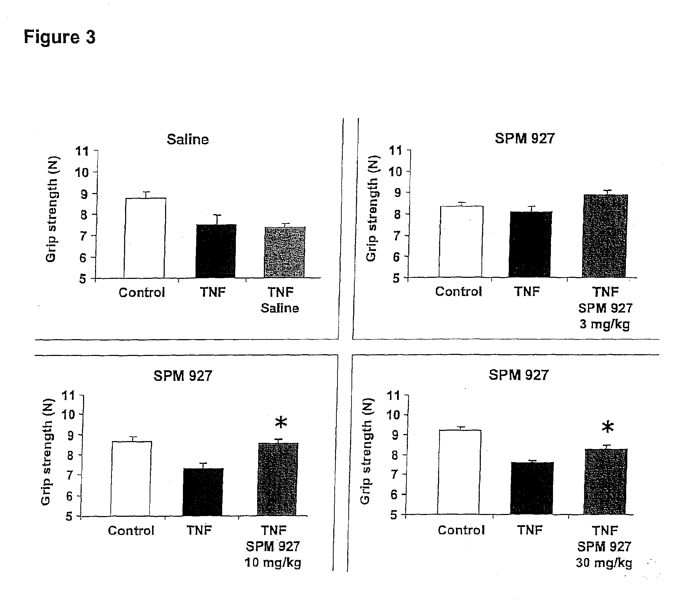

Figure 3 shows the absolute values of grip strength. A significant reversal of the TNF induced reduction of grip strength was seen with lacosamide at 10 and 30 mg/kg. A significant reversal was also seen for pregabalin at 100 mg/kg, gabapentin at 100 mg/kg and metamizol at 2 mg/kg.

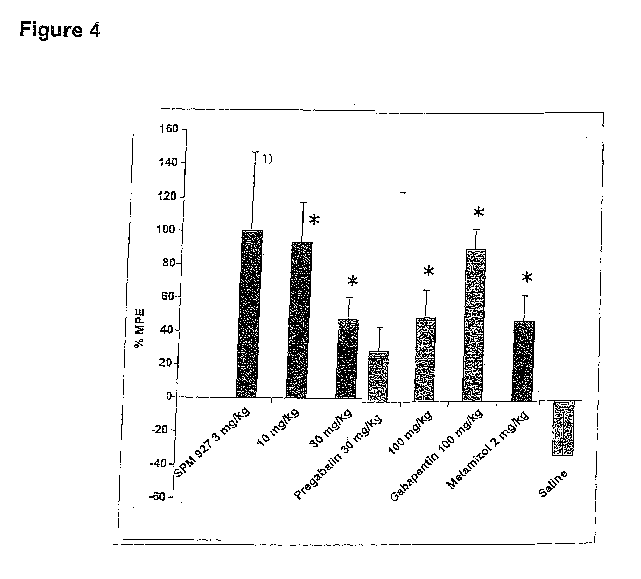

The MPE (Figure 4) was significantly different from vehicle for lacosamide at 10 and 30 mg/kg, for pregabalin at 100 mg/kg, for gabapentin at 100 mg/kg, and for metamizol at 2 mg/kg. The vehicles had no effect.

### Discussion

Withdrawal thresholds to pressure applied percutaneously to muscle were markedly reduced after TNF injection in most rats. This primary muscular hyperalgesia parallels tenderness to palpation that is observed clinically in patients with myalgia, such as myofascial pain syndrome, fibromyalgia and back pain (3). Tenderness to palpation is a primary criterion for the diagnosis of muscle pain under clinical and experimental human conditions (4,5).

Lacosamide dose dependently improves muscle hyperalgesia induced by TNF injection in the paw pressure test, reaching full reversal at 30 mg/kg. In comparison to the anticonvulsants pregabalin and gabapentin lacosamide has a stronger effect on muscle pain. Neither pregabalin nor gabapentin lead to a full reversal of the muscle hyperalgesia. In the grip strength test lacosamide reverses the effect of TNF on the muscle already at 10 mg/kg. Again lacosamide is more potent than pregabalin and gabapentin which improve grip strength only at 100 mg/kg.

In conclusion, lacosamide was effective in reducing the muscular hyperalgesia induced by TNF injected into muscle. Thus, lacosamide and related compounds as disclosed in formulae (Ib) and (IIb) have therapeutic efficacy in the treatment, in particular systemic treatment, of specific manifestations of non-inflammatory musculoskeletal pain such as muscular hyperalgesia and allodynia occurring in fibromyalgia, myofascial pain syndrome, back pain or osteoarthritis.

### Example 2:

**Study of antinociceptive effect of lacosamide in iodoacetate rat model.**

### Animals

Male Wistar rats (Janview, France) weighing 170-200 g at the start of the study were used. The animals were group-housed (3 animals per cage) in a room with controlled temperature (21-22°C), and a reversed light-dark cycle (12 h/12 h), and had free access to food and water.

### Induction of osteoarthritis

Osteoarthritis was induced by intra-articular injection in 50 µl of 3 mg monosodium iodoacetate (MIA) (Sigma) through the intrapatellar ligament of the right knee. Control rats were injected with an equivalent volume of saline. Up to five days after the iodoacetate injection a substantial inflammation of synovial joints was observed in this model. The general health of the animals was monitored. No signs of distress were seen.

### Histology

On each of days 3, 7 and 14 after iodoacetate treatment, four animals were sacrificed for histology study. Knees were harvested and fixed overnight in 10% formalin and subsequently decalcified with 10% formic acid for 72 h before being embedded in paraffin. Sections 10 µm thick were prepared every 250 µm. Hematoxylin/eosin staining was carried out to assess the extent of inflammatory infiltrates in the joints and surrounding tissues, and Saflanin-O fast green staining was done to measure the degeneration of cartilage.

### Evaluation of the effect of compounds on nociception

In the first round of experiments the iodoacetate-treated rats were randomized to six experimental groups (12 animals per group) which received the following treatments (p.o. = *per os*; s.c. = subcutaneous) on the days of pain assessment (days 3, 7 and 14 postiodoacetate treatment):
p.o. injection of saline (vehicle);
p.o. injection of 3 mg/kg lacosamide;
p.o. injection of 10 mg/kg lacosamide;
p.o. injection of 30 mg/kg lacosamide;
s.c. injection of 3 mg/kg morphine.

Diclofenac (30 mg/kg, s.c.) was tested in a separate experiment by the same scientists under the same conditions at about the same time. The non-iodoacetate treated control group (control) received p.o. injection of saline 45 minutes prior to the pain assessment. Lacosamide, diclofenac and morphine were injected 60 minutes prior to implementation of behavioral tests. Each group was examined blind.

### Evaluation of tactile allodynia and mechanical hyperalgesia

For testing tactile allodynia, rats were placed on a metallic grid floor. Nociceptive testing was done by inserting a von Frey filament (Bioseb, France) through the grid floor and applying it to the plantar surface of the hind paw. A trial consisted of several applications of different von Frey filaments (at a frequency of about 1 Hz). The von Frey filaments were applied from filament 10 g to 100 g. As soon as the animal removed its hind paw, the test was stopped and the filament number was recorded to represent the paw withdrawal threshold.

For testing mechanical hyperalgesia, nociceptive flexion reflexes were quantified using the Randall-Selitto paw pressure device (Bioseb, France), which applied a linearly increasing mechanical force to the dorsum of the rat's hind paw. The paw withdrawal threshold was defined as the force at which the rat withdrew its paw. The cutoff pressure was set to 250 g.

### Drugs and reagents

Lacosamide (Schwarz BioSciences GmbH) and morphine sulfate (Francopia, France) were dissolved in saline. Monosodium iodoacetate and diclofenac were purchased from Sigma (France). Drug administration was made in a volume of 1 ml/kg.

### Data analyses and statistics

Comparisons of groups of behavioral data at each individual time point were conducted using ANOVA followed by post-hoc analysis (Dunnett's test).

### Results

Joint pathology was assessed on day 3, 7 and 14 following intra-articular injection of iodoacetate. At day 3 there was a substantial initial inflammatory response. This inflammation was characterized by an expansion of the synovial membrane most likely caused by proteinaceous edema fluid and fibrin with infiltrating macrophages, neutrophils, plasma cells and lymphocytes. The cartilage was still intact. By day 7, inflammation within the synovium and surrounding tissue has largely resolved. On day 14 proteoglycan loss was seen throughout the depth of the cartilage. The synovial membrane looked normal and contained no inflammatory cells.

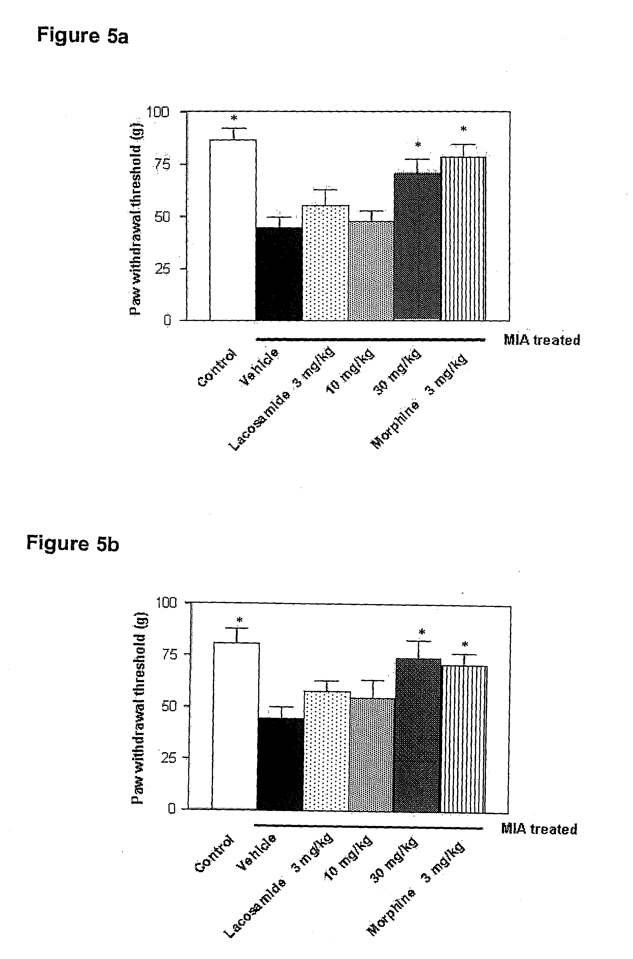

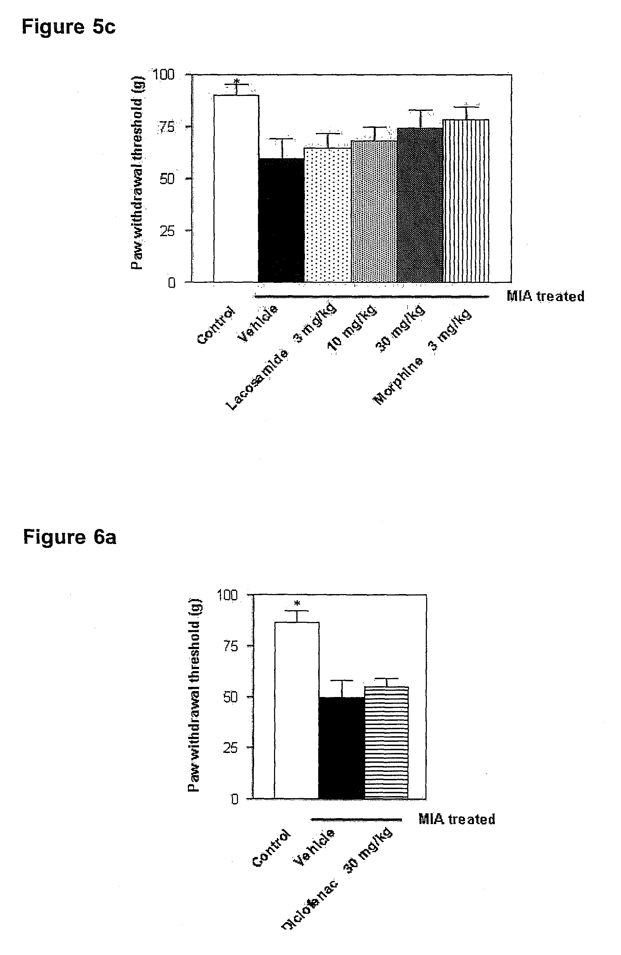

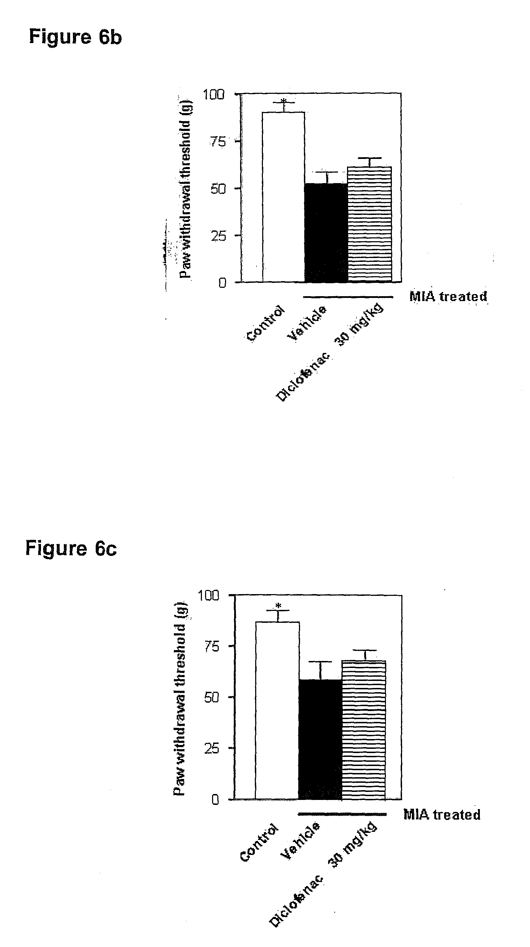

Tactile allodynia, tested with von Frey filaments, was assessed at day 3, 7, and 14 in iodoacetate-treated rats compared to control rats. Treatment with lacosamide (30 mg/kg) and morphine (3 mg/kg) improved tactile allodynia of iodoacetate-treated rats at day 3 (Fig. 5A) and 7 (Fig. 5B) but not on day 14 (Fig. 5C), and lower doses of lacosamide showed a trend for such improvement. Diclofenac (30 mg/kg) had no effect on tactile allodynia at day 3 (Fig. 6A), day 7 (Fig. 6B) or day 14 (Fig. 6C).

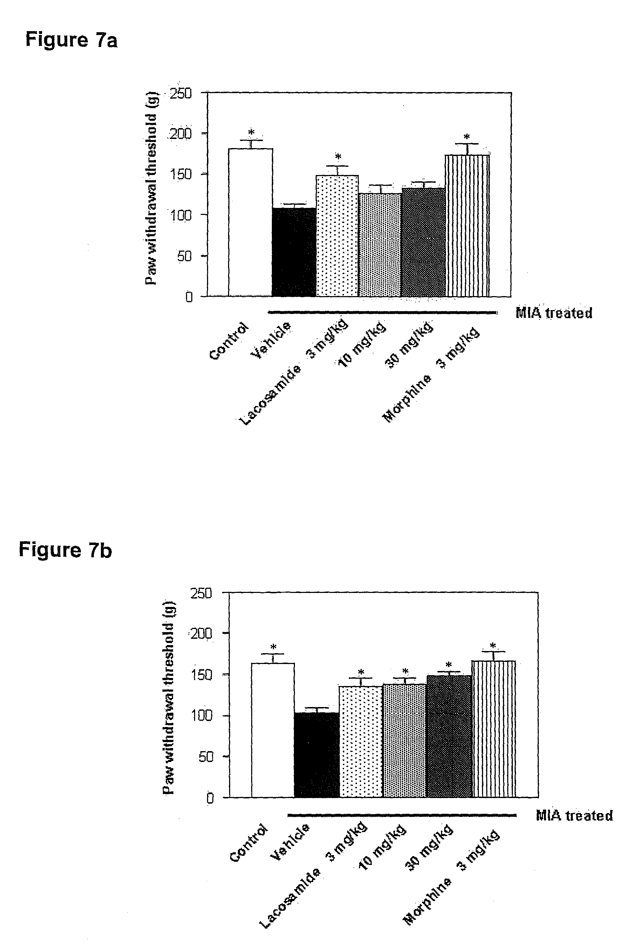

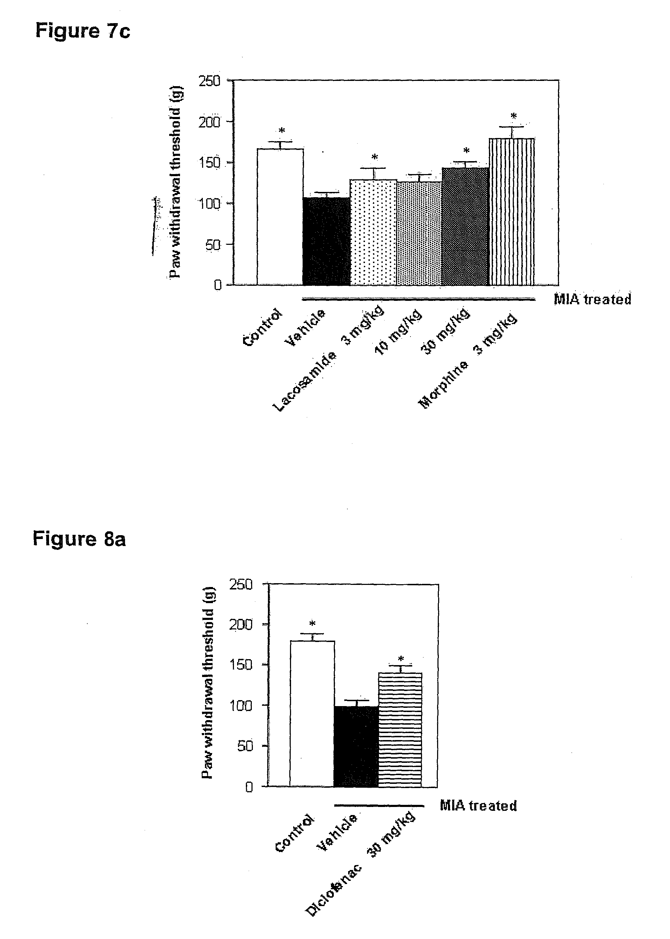

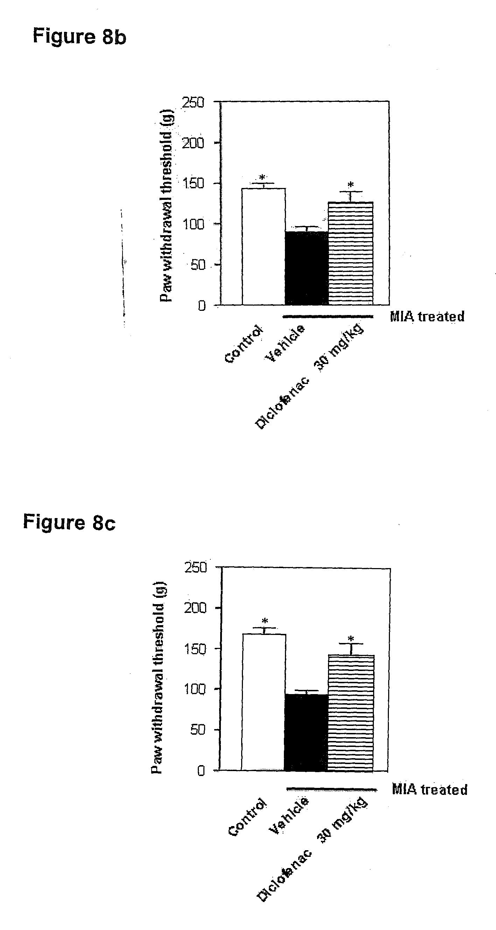

There was a marked mechanical hyperalgesia as evidenced by a reduction in the paw pressure withdrawal thresholds in the iodoacetate/vehicle treated animals compared to control/vehicle treated animals. Treatment of iodoacetate-treated rats with lacosamide 3 mg/kg, morphine 3 mg/kg and diclofenac 30 mg/kg induced in each case an increase in paw pressure withdrawal threshold compared to iodoacetate/vehicle treated animals on day 3 (Figs. 7A, 8A). On day 7, lacosamide at all doses tested (3, 10 and 30 mg/kg), morphine and diclofenac each reduced mechanical hyperalgesia (Figs. 7B, 8B). Similar results were seen at day 14 after iodoacetate treatment except that the group treated with 10 mg/kg lacosamide did not show a statistically significant effect (Figs. 7C, 8C). Interestingly, in the iodoacetate-treated animals, mechanical hyperalgesia developed from day 3 and lasted for at least 14 days, compared to tactile allodynia which was more pronounced during the early phase of osteoarthritis development, reflecting an ongoing development of pain sensitization based on different molecular mechanisms during the 14 days post iodoacetate-treatment.

The results show that lacosamide inhibited mechanical hyperalgesia during the post-inflammatory period, indicating effectiveness of lacosamide for treating noninflammatory osteoarthritic pain.

All patents and publications cited herein are incorporated by reference into this application in their entirety.

### References

1. Schäfers M, Sorkin LS, Sommer C. Intramuscular injection of tumor necrosis factor-alpha induces muscle hyperalgesia in rats. Pain 2003; 104(3):579-588.
2. Pongratz DE, Späth M. Morphologic aspects of fibromyalgia. Z Rheumatol 1998; 57 Suppl 2:47-51.
3. McCain GA. Fibromyalgia and myofascial pain. In: PD Wall and R Melzack (Eds.). Textbook of pain: Churchill Livingstone, New York, 1994. pp. 475-493.
4. Wolfe F, Smythe HA, Yunus MB, Bennett RM, Bombardier C, Goldenberg DL. The American College of Rheumatology 1990 criteria fo the classification of fibromyalgia: report of the multicenter criteria committee. Arthritis Rheum 1990; 33:160-172.
5. Arendt-Nielsen L. Induction and assessment of experimental pain from human skin, muscle, and viscera. In: TS Jensen, JA Turner and Z Wiesenfeld-Hallin (Eds.). Proceedings of the 8th World Congress of Pain: IASP Press, Seattle, 1997.
6. Nordenskiold UM, Grimby G. Grip force in patients with rheumatoid arthritis and fibromyalgia and in healthy subjects. A study with the Grippit instrument. Scand J Rheumatol 1993; 22:14-9.
7. Kniffki KD, Mense S, Schmidt RF. Responses of group IV afferent units from skeletal muscle to stretch, contraction and chemical stimulation. Exp Brain Res 1978; 31:511-22.
8. Mense S, Skeppar P. Discharge behaviour of feline gamma-motoneurones following induction of an artificial myositis. Pain 1991; 46:201-10.
9. Gur A, Karakoc M, Nas K, Remzi, Cevik, Denli A, et al. Cytokines and depression in cases with fibromyalgia. J Rheumatol 2002; 29:358-61.
10. Nampiaparampil DE, Shmerling RH (2004) A review of fibromyalgia. Am. J. Manag. Care 10:794-800.
11. Guzman R.E., Evans M.G., Bove S., Morenko B., Kilgore K., 2003. Mono-iodoacetate-induced histologic changes in subchondral bone and articular cartilage of rat femorotibial joints: an animal model of osteoarthritis. Toxicol Pathol. 31 (6), 619-624.
12. Kalbhen D.A., 1987. Chemical model of osteoarthritis--a pharmacological evaluation. J Rheumatol. 14 Spec No:130-131.
13. Wieland, H.A., Michaelis, M., Kirschbaum, B.J., Rudolphi, K.A. 2005. Osteoarthritis - an untreatable disease? Nature Reviews Drug Discovery 4,331-344.
14. Heppelmann B., 1997. Anatomy and histology of joint innervation. J Peripher Nerv Syst. 2(1), 5-16.
15. Mach D.B., Rogers S.D., Sabino M.C., Luger N.M., Schwei M.J., Pomonis J.D., Keyser C.P., Clohisy D.R., Adams D.J., O'Leary P., Mantyh P.W., 2002. Origins of skeletal pain: sensory and sympathetic innervation of the mouse femur. Neuroscience 113(1), 155-166.
16. Schaible H.G., Ebersberger A., Von Banchet G.S., 2002. Mechanisms of pain in arthritis. Ann N Y Acad Sci. 966, 343-354.
17. Bialer M., Johannessen, S.I., Kupferberg, H.J., Levy, R.H., Loiseau, P., Perucca, E., 2002, Progress report on new antiepileptic drugs: a summary of the Sixth Eilat Conference (EILAT VI). Epilepsy Res 51, 31-71.
18. Staud R., Smitherman, L. 2002. Peripheral and central sensitization in fibromyalgia: pathogenetic role. Current pain and headache reports 6, 259-266.

### The following items are part of the description

1. Use of a compound having the Formula (Ib) 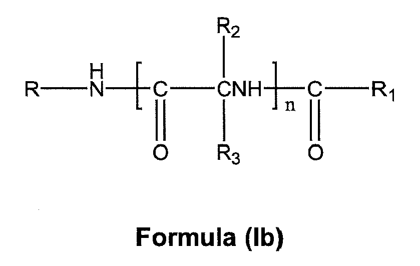 wherein
   R is hydrogen, lower alkyl, lower alkenyl, lower alkynyl, aryl, aryl lower alkyl, heterocyclic, heterocyclic lower alkyl, lower alkyl heterocyclic, lower cycloalkyl or lower cycloalkyl lower alkyl, and R is unsubstituted or is substituted with at least one electron withdrawing group or/and at least one electron donating group;
   R₁ is hydrogen or lower alkyl, lower alkenyl, lower alkynyl, aryl lower alkyl, aryl, heterocyclic lower alkyl, lower alkyl heterocyclic, heterocyclic, lower cycloalkyl, lower cycloalkyl lower alkyl, each unsubstituted or substituted with at least one electron donating group or/and at least one electron withdrawing group;
   R₂ and R₃ are independently hydrogen, lower alkyl, lower alkenyl, lower alkynyl, aryl lower alkyl, aryl, halo, heterocyclic, heterocyclic lower alkyl, lower alkyl heterocyclic, lower cycloalkyl, lower cycloalkyl lower alkyl, or Z-Y wherein R₂ and R₃ may be unsubstituted or substituted with at least one electron withdrawing group or/and at least one electron donating group; and wherein heterocyclic in R₂ and R₃ is furyl, thienyl, pyrazolyl, pyrrolyl, methylpyrrolyl, imidazolyl, indolyl, thiazolyl, oxazolyl, isothiazolyl, isoxazolyl, piperidyl, pyrrolinyl, piperazinyl, quinolyl, triazolyl, tetrazolyl, isoquinolyl, benzofuryl, benzothienyl, morpholinyl, benzoxazolyl, tetrahydrofuryl, pyranyl, indazolyl, purinyl, indolinyl, pyrazolindinyl, imidazolinyl, imidazolindinyl, pyrrolidinyl, furazanyl, N-methylindolyl, methylfuryl, pyridazinyl, pyrimidinyl, pyrazinyl, pyridyl, epoxy, aziridino, oxetanyl, azetidinyl or, when N is present in the heterocyclic, an N-oxide thereof;
   Z is O, S, S(O)ₐ, NR₄, NR₆' or PR₄ or a chemical bond;
   Y is hydrogen, lower alkyl, aryl, aryl lower alkyl, lower alkenyl, lower alkynyl, halo, heterocyclic, heterocyclic lower alkyl, lower alkyl heterocyclic and Y may be unsubstituted or substituted with at least one electron donating group or/and at least one an electron withdrawing group, wherein heterocyclic has the same meaning as in R₂ or R₃ and, provided that when Y is halo, Z is a chemical bond, or ZY taken together is NR₄NR₅R₇, NR₄OR₅, ONR₄R₇, OPR₄R₅, PR₄OR₅, SNR₄R₇, NR₄SR₇, SPR₄R₅, PR₄SR₇, NR₄PR₅R₆, PR₄NR₅R₇, or N⁺R₅R₆R₇, 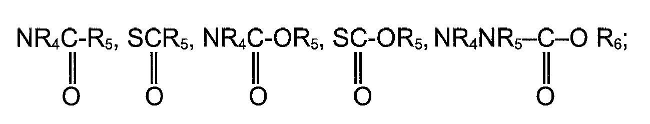 R₆' is hydrogen, lower alkyl, lower alkenyl, or lower alkynyl which may be unsubstituted or substituted with at least one electron withdrawing group or/and at least one electron donating group;
   R₄, R₅ and R₆ are independently hydrogen, lower alkyl, aryl, aryl lower alkyl, lower alkenyl, or lower alkynyl, wherein R₄, R₅ and R₆ may independently be unsubstituted or substituted with at least one electron withdrawing group or/and at least one electron donating group; and
   R₇ is R₆ or COOR₈ or COR₈, which R₇ may be unsubstituted or substituted with at least one electron withdrawing group or/and at least one electron donating group;
   R₈ is hydrogen or lower alkyl, or aryl lower alkyl, and the aryl or alkyl group may be unsubstituted or substituted with at least one electron withdrawing group or/and at least one electron donating group; and n is 1-4; and
   a is 1-3,
   or of a pharmaceutically acceptable salt thereof,
   for the preparation of a pharmaceutical composition for the prevention, alleviation or/and treatment of non-inflammatory musculoskeletal pain or/and non-inflammatory osteoarthritic pain, such as muscular hyperalgesia or/and allodynia occurring in fibromyalgia, myofascial pain syndrome, back pain or/and osteoarthritis.
2. Use according to item 1, wherein the non-inflammatory musculoskeletal pain is non-inflammatory musculoskeletal pain or/and non-inflammatory osteoarthritic pain associated with or/and caused by a pathological condition selected from regional pain syndrome such as back or neck pain, rheumatoid arthritis, osteoarthritis, gout, ankylosing spondylitis, lupus erythematosus, fibromyalgia, fibrositis, fibromyositis, myofascial pain syndrome, autoimmune disorders, polymyalgia rheumatica, polymyositis, dermatomyositis, muscular abscess, trichinosis, Lyme disease, Malaria, Rocky Mountain spotted fever, polio, trauma, joint damage, joint damage by trauma, cartilage degradation, structural bone changes, and vascularization of areas of osteoarthritic bone remodeling.
3. Use according to item 1 or 2, wherein the non-inflammatory osteoarthritic pain is non-inflammatory osteoarthritic pain associated with or/and caused by a pathological condition selected from trauma, joint damage, joint damage by trauma, cartilage degradation, structural bone changes, and vascularization of areas of osteoarthritic bone remodeling.
4. Use according to any of the items 1 to 3, wherein the non-inflammatory musculoskeletal pain or/and non-inflammatory osteoarthritic pain is characterized by the absence of swelling or warmth, absence of inflammatory or/and systemic features, or/and essentially no morning stiffness.
5. Use according to any of the items 1 to 4, wherein non-inflammatory musculoskeletal pain or/and osteoarthritic pain includes a condition associated with or/and caused by non-inflammatory musculoskeletal pain or/and non-inflammatory osteoarthritic pain selected from fatigue, sleep disorder, irritable bowel syndrome, chronic headache, temporo-mandibular joint dysfunction syndrome, multiple chemical sensitivity, painful menstrual periods, dysmenorrhea, chest pain, morning stiffness, cognitive or memory impairment, numbness and tingling sensations, muscle twitching, irritable bladder, the feeling of swollen extremities, skin sensitivities, dry eyes and mouth, frequent changes in eye prescription, dizziness, and impaired coordination.
6. Use according to any of items 1 to 5, wherein the non-inflammatory musculoskeletal pain is non-inflammatory pain associated with or/and caused by osteoarthritis, in particular non-inflammatory musculoskeletal pain associated with or/and caused by osteoarthritis.
7. Use according to item 6, wherein the non-inflammatory pain is non-inflammatory osteoarthritic pain.
8. Use according to any one of items 1-7 wherein one of R₂ and R₃ is hydrogen.
9. Use according to any one of items 1-8 wherein n is 1.
10. Use according to any one of items 1-9 wherein one of R₂ and R₃ is hydrogen and n is 1.
11. Use according to any one of items 1-10 wherein R is aryl lower alkyl and R₁ is lower alkyl.
12. Use according to any one of items 1-11 wherein
   R₂ and R₃ are independently hydrogen, lower alkyl, or ZY;
   Z is O, NR₄ or PR₄;
   Y is hydrogen or lower alkyl or
   ZY is NR₄NR₅R₇, NR₄OR₅, ONR₄R₇, 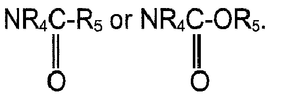
13. Use according to item 12 wherein R₂ is hydrogen and and R₃ is lower alkyl, or ZY;
   Z is O, NR₄ or PR₄;
   Y is hydrogen or lower alkyl;
   ZY is NR₄NR₅R₇, NR₄OR₅, 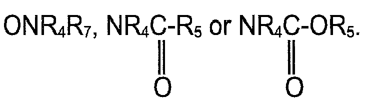
14. Use according any one of items 1-13 wherein R₂ is hydrogen and R₃ is lower alkyl, which may be substituted or unsubstituted with at least one electron donating group or/and at least one electron withdrawing group, NR₄OR₅, or ONR₄R₇.
15. Use according to any one of items 1-14 wherein R₃ is lower alkyl which is unsubstituted or substituted with hydroxy or lower alkoxy, NR₄OR₅ or ONR₄R₇, wherein R₄, R₅ and R₇ are independently hydrogen or lower alkyl, R is aryl lower alkyl, which aryl group may be unsubstituted or substituted with at least one electron withdrawing group and R₁ is lower alkyl.
16. Use according to any one of items 1-15 wherein aryl is phenyl and is unsubstituted or substituted with halo.
17. Use according to any of items 1-14 wherein the compound is
   (R)-2-acetamido-N-benzyl-3-methoxy-propionamide;
   (R)-2-acetamido-N-benzyl-3-ethoxy-propionamide;
   O-methyl-N-acetyl-D-serine-m-fluorobenzylamide;
   O-methyl-N-acetyl-D-serine-p-fluorobenzylamide;
   N-acetyl-D-phenylglycinebenzylamide;
   D-1,2-(N, O-dimethylhydroxylamino)-2-acetamide acetic acid benzylamide; or
   D-1,2-(O-methylhydroxylamino)-2-acetamido acetic acid benzylamide.
18. Use of any one of items 1-16 wherein the compound has the Formula (IIb) 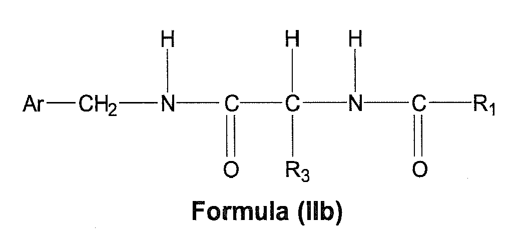 wherein
   Ar is phenyl which is unsubstituted or substituted with at least one halo group;
   R₃ is CH₂-Q, wherein Q is lower alkoxy containing 1-3 carbon atoms and R₁ is lower alkyl containing 1-3 carbon atoms
   or of a pharmaceutically acceptable salt thereof.
19. Use according to item 18 wherein Ar is unsubstituted phenyl.
20. Use according to items 18 wherein halo is fluoro.
21. Use according to items 18 to 19 wherein R₃ is CH₂-Q, wherein Q is alkoxy containing 1-3 carbon atoms and Ar is unsubstituted phenyl.
22. Use of any one of items 1-16 wherein the compound is in the R configuration and has the formula 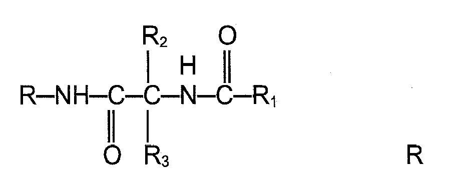 wherein
   R is benzyl which is unsubstituted or substituted with at least one halo group;
   R₃ is CH₂-Q, wherein Q is lower alkoxy containing 1-3 carbon atoms and R₁ is methyl
   or a pharmaceutically acceptable salt thereof.
23. Use according to item 22 wherein the compound is substantially enantiopure.
24. Use according to items 22 or 23 wherein R is unsubstituted benzyl.
25. Use according to items 22 to 23 wherein halo is fluoro.
26. Use according to items 22 to 24 wherein R₃ is CH₂-Q, wherein Q is alkoxy containing 1-3 carbon atoms and R is unsubstituted benzyl.
27. Use according to any of the items 1 to 7, wherein the compound of Formula (Ib) is (R)-2-Acetamido-N-benzyl-3-methoxypropionamide or a pharmaceutically acceptable salt thereof.
28. Use according to item 27 wherein the compound is substantially enantiopure.
29. Use according to any one of the preceding items, wherein the pharmaceutical composition is prepared for treatment with doses of the compound at least of 100 mg/day, preferably at least of 200 mg/day, more preferably at least of 300 mg/day, most preferably at least of 400 ,mg/day.
30. Use according to any one of the preceding items, wherein the pharmaceutical composition is prepared for treatment with doses of the compound at a maximum of 6 g/day, more preferably at a maximum of 1 g/day and most preferably at a maximum of 600 mg/day.
31. Use according to any one of the preceding items, wherein the pharmaceutical composition is prepared for treatment with increasing daily doses until a predetermined daily dose is reached which is maintained during the further treatment.
32. Use according to any one of the preceding items, wherein the pharmaceutical composition is prepared for treatment in three doses per day, preferably two doses per day, more preferably in a single dose per day.
33. Use according to any one of the preceding items, wherein the pharmaceutical composition is prepared for an administration resulting in a plasma concentration of 0.1 to 15 µg/ml (trough) and 5 to 18.5 µg/ml (peak), calculated as an average over a plurality of treated subjects.
34. Use according to any one of the preceding items, wherein the pharmaceutical composition is prepared for oral or i.v. administration.
35. Use according to any one of the preceding items, wherein the pharmaceutical composition further comprises an active agent for the prevention, alleviation or/and treatment of non-inflammatory musculoskeletal pain or/and non-inflammatory osteoarthritic pain such as muscular hyperalgesia or/and allodynia occurring in fibromyalgia, myofascial pain syndrome, back pain or/and osteoarthritis.
36. Use according to item 35 wherein the pharmaceutical composition comprises a single dose form or comprises a separate dose form comprising a first composition comprising a compound as defined in any of the items 1 and 8 to 28 and a second composition comprising the further active agent.
37. Use according to any one of the preceding items wherein the pharmaceutical composition is prepared for administration in mammals.
38. Use according to item 37 wherein the pharmaceutical composition is prepared for administration in humans.
39. A pharmaceutical composition comprising
   (a) a compound as defined in any of the items 1 and 8 to 28, and
   (b) a further active agent for the prevention, alleviation or/and treatment of non-inflammatory musculoskeletal pain or/and non-inflammatory osteoarthritic pain such as muscular hyperalgesia or/and allodynia occurring in fibromyalgia, myofascial pain syndrome, back pain or/and osteoarthritis, in particular a compound different from those of (a).
40. The pharmaceutical composition according to item 39, wherein the compound of (a) is (R)-2-acetamide-N-benzyl-3-methoxypropionamide.
41. The pharmaceutical composition according to item 39 or 40 which comprises a single dose form or a separate dose form comprising a first composition comprising a compound as defined in any of the items 1 and 8 to 28 and a second composition comprising the further active agent (b).
42. The pharmaceutical composition of any of the items 39 to 41, wherein the further active agent is an anticonvulsant.
43. The pharmaceutical composition of item 42, wherein the anticonvulsant is selected from the group consisting of carbamazepine, phenytoin, gabapentin, pregabalin, lamotrigine, and levetiracetam.
44. The pharmaceutical composition of any of the items 39 to 41 wherein the further acitve agent is at least one anti-osteoarthritis agent other than an anticonvulsant.
45. The pharmaceutical composition of any of the items 39 to 41 and 44, wherein the further acitve agent, in particular the anti-osteoarthritis agent is an opioid or non-opioid analgesic, a steroidal anti-inflammatory, an NSAID or COX-2 selective inhibitor, or a DMOAD.
46. A pharmaceutical composition comprising
   (a) a compound as defined in any of the items 1 and 8 to 28, and
   (b) a further active agent for the prevention, alleviation or/and treatment of non-inflammatory arthritic pain, such as pain associated with or/and caused by osteoarthritis.
47. The pharmaceutical composition according to item 46, wherein the compound of (a) is (R)-2-acetamide-N-benzyl-3-methoxypropionamide.

## Claims

1. A pharmaceutical composition or combination comprising
(a) a compound having the Formula (IIb) 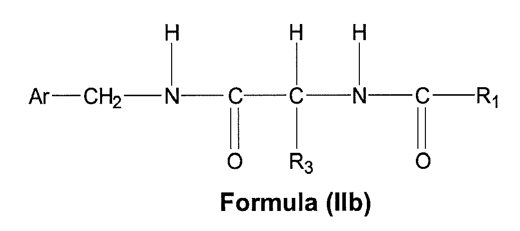 wherein
Ar is phenyl which is unsubstituted or substituted with at least one halo group,
R₃ is CH₂-Q, wherein Q is alkoxy containing 1-3 carbon atoms and R₁ is alkyl containing 1-3 carbon atoms,
or a pharmaceutically acceptable salts thereof,
and
(b) an anticonvulsant selected from the group consisting of carbamazepine, gabapentin, pregabalin, lamotrigine, and levetiracetam.

2. The pharmaceutical composition or combination according to claim 1, wherein Ar is unsubstituted phenyl.

3. The pharmaceutical composition or combination according to claim 1, wherein Ar is substituted phenyl and halo is fluoro.

4. The pharmaceutical composition or combination according to claim 1, wherein the compound of formula (IIb) is (R)-2-acetamido-N-benzyl-3-methoxy-propionamide; (R)-2-acetamido-N-benzyl-3-ethoxy-propionamide; O-methyl-N-acetyl-D-serine-m-fluorobenzylamide; or O-methyl-N-acetyl-D-serine-p-fluorobenzylamide.

5. The pharmaceutical composition or combination according to claim 1, wherein the compound of (a) is (R)-2-acetamido-N-benzyl-3-methoxypropionamide or a pharmaceutically acceptable salt thereof.

6. The pharmaceutical composition or combination according to claim 1, wherein the compound of (a) is (R)-2-acetamido-N-benzyl-3-methoxypropionamide.

7. The pharmaceutical composition or combination according to any one of the claims 1 to 6, wherein the compound of (b) is carbamazepine.

8. The pharmaceutical composition or combination according to any one of the claims 1 to 6, wherein the compound of (b) is gabapentin.

9. The pharmaceutical composition or combination according to any one of the claims 1 to 6, wherein the compound of (b) is pregabalin.

10. The pharmaceutical composition or combination according to any one of the claims 1 to 6, wherein the compound of (b) is levetiracetam.

11. The pharmaceutical composition or combination according to any one of the claims 1 to 10 which comprises a single dose form.

12. The pharmaceutical composition or combination according to any one of the claims 1 to 10 which comprises a separate dose form comprising
(i) a first composition comprising the compound (a), and
(ii) a second composition comprising the anticonvulsant (b).

13. The pharmaceutical composition or combination according to any one of the preceding claims prepared for treatment with doses of the compound at least of 100 mg/day, preferably at least of 200 mg/day.

14. The pharmaceutical composition or combination according to any one of the preceding claims prepared for treatment with doses of the compound at a maximum of 1 g/day and preferably at a maximum of 600 mg/day.

15. The pharmaceutical composition or combination according to any one of the preceding claims prepared for treatment in three doses per day, preferably two doses per day.

16. The pharmaceutical composition or combination according to any one of the preceding claims prepared for an administration resulting in a plasma concentration of 0.1 to 15 µg/ml (trough) and 5 to 18.5 µg/ml (peak), calculated as an average over a plurality of treated subjects.

17. The pharmaceutical composition or combination according to any one of the preceding claims prepared for oral or i.v. administration.

18. The pharmaceutical composition or combination according to any one of the preceding claims prepared for administration in mammals or/and in humans.
